# EUROPEAN PATENT APPLICATION

(11) **EP 4 501 948 A1**
(43) Date of publication of application: **05.02.2025**
(21) Application number: 23775097.1
(22) Date of filing: 24.03.2023
(51) Int. Cl.: C07K 14/31, C07K 14/195

(54) **SET OF AFFIBODY AND PHOTORECEPTOR PROTEIN**

(30) Priority: 24.03.2022 JP 2022048984
(71) Applicant: The University of Tokyo, Bunkyo-ku, Tokyo 113-8654 (JP); KANAGAWA INSTITUTE OF INDUSTRIAL SCIENCE AND TECHNOLOGY, Ebina-shi Kanagawa 243-0435 (JP)
(72) Inventor: SATO, Moritoshi, Tokyo 113-8654 (JP); KUWASAKI, Yuto, Tokyo 113-8654 (JP); NAKAJIMA, Takahiro, Ebina-shi, Kanagawa 243-0435 (JP)
(74) Representative: Mewburn Ellis LLP
(86) International application number: PCT/JP2023/011866
(87) International publication number: WO 2023/182504

(57) **Abstract**

The present invention provides a set of Affibody and photoreceptor protein, wherein the Affibody and the photoreceptor protein form a complex in a manner dependent on light at a wavelength of 600 to 750 nm.

## Description

### Technical Field

The present invention relates to a set of Affibody and photoreceptor protein. The present invention also relates to a genetic modification technique using the set of Affibody and photoreceptor protein.

### Background Art

A molecular control approach that exploits protein photoactivation has emerged and is called optogenetics. Light is easily controlled in terms of space or time as compared with chemicals. A light-dependent protein-protein interaction system is known which exploits photosensitive protein that forms a dimer in a light-dependent manner or a photoreceptor and its binding partner.

Patent Literature 1 discloses a set of proteins useful as a tool of optogenetics, the set being capable of controlling dimer formation and dissociation by light irradiation, as an optical manipulation technique using blue light.

Non Patent Literatures 1 to 3 have also been reported as manipulation techniques using red light.

### Citation List

### Patent Literature

Patent Literature 1: Japanese Patent Laid-Open No. 2015-165776

### Non Patent Literature

Non Patent Literature 1: Nature Biotechnology, 2002, 20, 1041-1044
Non Patent Literature 2: Nature Methods, 2016, 13, 591-597
Non Patent Literature 3: ACS Synth. Biol. 2020, 9, 3322-3333

### Summary of Invention

### Technical Problem

Although optical manipulation techniques using red light are known, the existence of many problems is known such as a need to add a cofactor, difficult manipulation using light at two different long wavelengths, and difficulty in precise manipulation. There exist other problems including the inability to apply red light to the optical manipulation of various proteins.

An object of the present invention is to develop a photoswitching protein that can optically manipulate the functions of various proteins with precision and high efficiency by light at two different long wavelengths without the use of blue light.

### Solution to Problem

The present inventors have conducted diligent studies to attain the object and consequently completed the present invention by finding that a set of Affibody and photoreceptor protein is optically controllable by red light.

The present invention is as follows:
[1] A set of Affibody and photoreceptor protein, wherein the Affibody and the photoreceptor protein form a complex in a manner dependent on light at a wavelength of 600 to 750 nm.
[2] The set according to [1], wherein the Affibody has any of the following amino acid sequences (1) to (4):
   (1) the amino acid sequence represented by SEQ ID NO: 1,
   (2) an amino acid sequence differing from the amino acid sequence represented by SEQ ID NO: 1 by mutation of an amino acid at one to five positions among positions 9 to 11, 13, 14, 17, 18, 24, 25, 27, 28, 32, and 35,
   (3) an amino acid sequence having 80% or higher homology to the portions of the amino acid sequence represented by SEQ ID NO: 1, outside of the positions 9 to 11, 13, 14, 17, 18, 24, 25, 27, 28, 32, and 35, and
   (4) an amino acid sequence derived from the amino acid sequence represented by SEQ ID NO: 1, having mutation of an amino acid at one to five positions among positions 9 to 11, 13, 14, 17, 18, 24, 25, 27, 28, 32, and 35 and having 80% or higher homology to the portions of the amino acid sequence represented by SEQ ID NO: 1, outside of the positions 9 to 11, 13, 14, 17, 18, 24, 25, 27, 28, 32, and 35.
[3] The set according to [1], wherein the Affibody has the following amino acid sequence (5) or (6):
   (5) an amino acid sequence differing from the amino acid sequence represented by SEQ ID NO: 1 by mutation of an amino acid at position 18 and/or 24, and
   (6) an amino acid sequence derived from the amino acid sequence represented by SEQ ID NO: 1, having mutation of an amino acid at position 18 and/or 24 and having 80% or higher homology to the portions of the amino acid sequence represented by SEQ ID NO: 1, outside of the positions 18 and 24.
[4] The set according to [1], wherein the Affibody has the following amino acid sequence (7) or (8):
   (7) an amino acid sequence differing from the amino acid sequence represented by SEQ ID NO: 1 by having any amino acid selected from phenylalanine (F), tryptophan (W), histidine (H), and methionine (M) at position 18, and
   (8) an amino acid sequence derived from the amino acid sequence represented by SEQ ID NO: 1, having any amino acid selected from phenylalanine (F), tryptophan (W), histidine (H), and methionine (M) at position 18 and having 80% or higher homology to the portions of the amino acid sequence represented by SEQ ID NO: 1, outside of the position 18.
[5] The set according to [1], wherein the Affibody has the following amino acid sequence (9):
   (9) an amino acid sequence differing from the amino acid sequence represented by SEQ ID NO: 1 by having any amino acid selected from phenylalanine (F), tryptophan (W), histidine (H), and methionine (M) at position 18 and lacking one to five N-terminal or C-terminal amino acids of SEQ ID NO: 1.
[6] The set according to any of [1] to [5], wherein the photoreceptor protein is DrBphP.
[7] The set according to any of [1] to [6], wherein the photoreceptor protein
   (1) has the amino acid sequence represented by SEQ ID NO: 2, or
   (2) has an amino acid sequence having 80% or higher homology to the amino acid sequence represented by SEQ ID NO: 2.
[8] The set according to any of [1] to [7], wherein a domain related to the transcriptional activity of a gene is bound to one of the Affibody and the photoreceptor protein, and a nucleic acid recognition domain is bound to the other.
[9] The set according to any of [1] to [8], wherein one of two fragments into which a protein having a predetermined effect is split is bound to the Affibody, and the other of the two fragments into which the protein having a predetermined effect is split is bound to the photoreceptor protein.

### Advantageous Effects of Invention

The present invention can provide a photoswitching protein that can optically manipulate the functions of various proteins with precision and high efficiency by light at two different long wavelengths without the use of blue light.

### Brief Description of Drawings

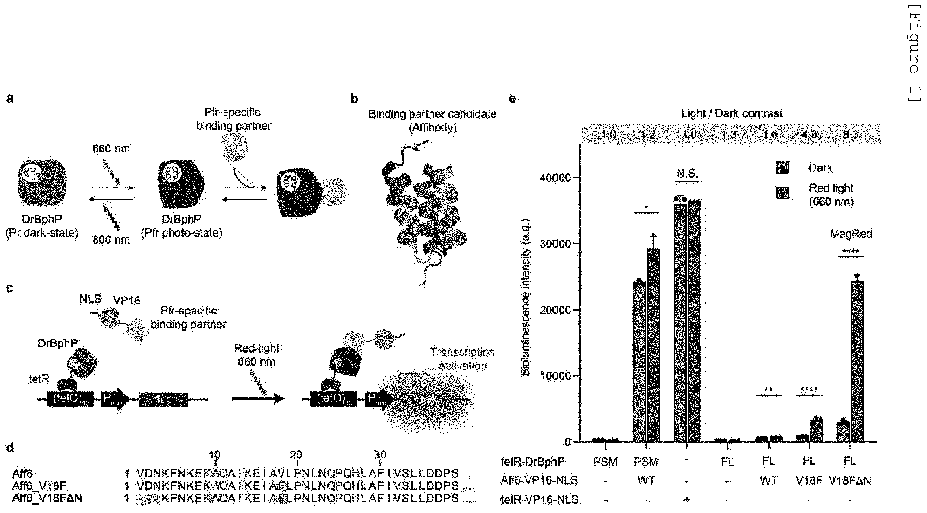

[Figure 1] Figure 1 illustrates MagRed. MagRed means a combination of photoreceptor protein such as DrBphP and Affibody. In the drawing, use of DrBphP as an example of the photoreceptor protein is illustrated. In the drawing, the Affibody is described as a Pfr specific biding partner. Figure 1a shows a schematic view of DrBphP and its photo-state (Pfr) form-specific Affibody. Figure 1b shows a diagram in which residues randomizable to the crystal structure (PDB: 2M5A) of the Affibody are drawn in a ball filling mode. Figure 1c shows a schematic view of a gene expression system based on a tetR-tetO system using DrBphP and its photo-state (Pfr) form-specific Affibody. Upon irradiation with light at 660 nm, Affibody linked to VP16 and a nuclear localization sequence (NLS) binds to tetR-DrBphP bound to a tetO sequence to activate the transcription of a reporter (fluc: firefly luciferase) (Pmin: minimal cytomegalovirus promoter). Figure 1d shows the alignment of N-terminal amino acid sequences of Affibody (Aff6) acquired by screening and its mutants (Aff6_V18F and Aff6_V18FΔN). Partial sequences of the amino acid sequences of the Affibody molecules designated as Aff6, Aff6_V18F, and Aff6_V18FΔN are shown (SEQ ID NOs: 63 to 65). Figure 1e shows results of comparing the bioluminescence intensity as an index of HeLa cells harboring a bioluminescent reporter plasmid by combining transcriptional activators (Aff6-VP16-NLS) respectively containing Affibody (Aff6) acquired by screening and its mutants (Aff6_V18F and Aff6_V18FΔN) with full-length (FL) DrBphP.

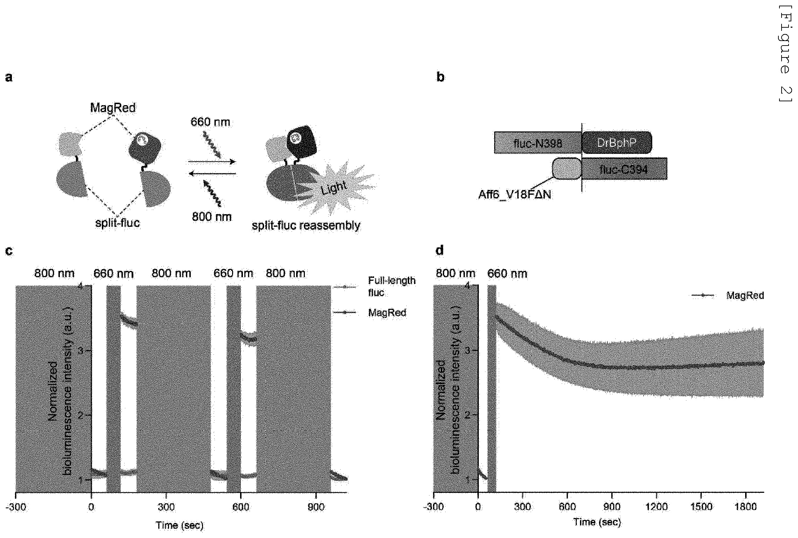

[Figure 2] Figure 2 shows results of evaluating the binding and dissociation of MagRed by split luciferase assay. Figure 2a shows a schematic view of the split luciferase assay. Upon irradiation with light at 660 nm, both fragments (fluc-N398 and fluc-C394) of split luciferase fused with MagRed (DrBphP and Aff6_V18FΔN) bind to each other to increase bioluminescent signals. Upon irradiation with light at 800 nm, both the fragments of the split luciferase fused with MagRed dissociate to decrease bioluminescent signals. Figure 2b shows a schematic view of the domain structure of the fusion protein used in the split luciferase assay. Figure 2c shows time courses of change in the bioluminescence of the split luciferase fused with MagRed and the bioluminescence of full-length luciferase (flue) when light irradiation at 660 nm (10 W/m²) for 1 minute and light irradiation at 800 nm (50 W/m²) for 5 minutes were repeated. Bioluminescent signals were normalized with minimal intensity in each sample. Figure 2d shows a time course of bioluminescence from the split luciferase for 30 minutes from the termination of light irradiation (10 W/m²) at 660 nm. Bioluminescent signals were normalized with minimal intensity in each sample.

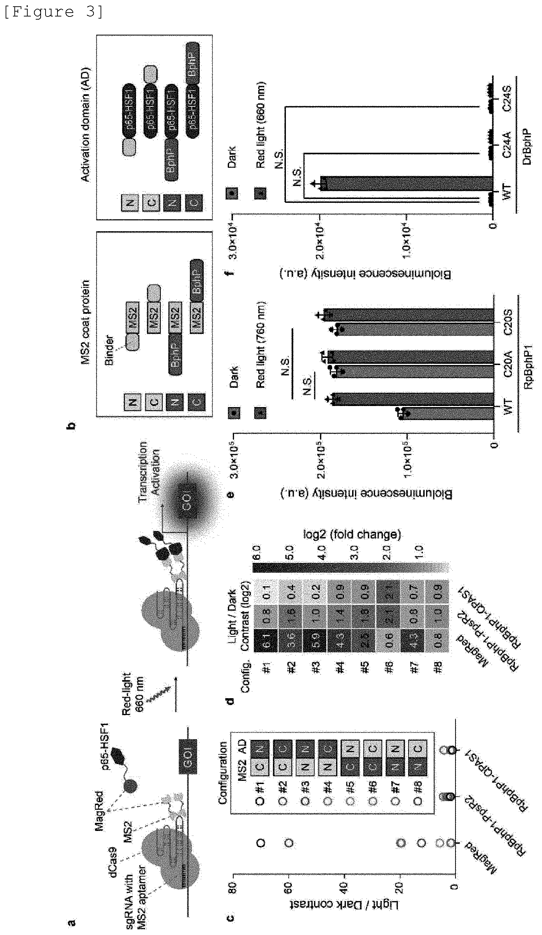

[Figure 3] Figure 3 shows results of comparing MagRed and RpBphP1-PpsR2/QPAS1 in an optical manipulation system (CPTS) of gene expression using CRISPR-Cas9. Figure 3a shows a schematic view of Red-CPTS using MagRed. dCas9 binds to guide RNA (sgRNA) containing MS2-RNA aptamer and binds to genomic DNA. The proteins (DrBphP and Aff6_V18FΔN) constituting MagRed were fused with a transcriptional activation domain (p65-HSF1) and MS2 protein. Upon irradiation with light at 660 nm, p65-HSF1 was recruited to target loci through the function of MagRed to activate the transcription of the target gene. Figures 3b, 3c, and 3d show comprehensive configuration of CPTS in which BphP or its binding partner was fused with the N or C terminus of a transcriptional activation domain (p65-HSF1) or MS2 protein. Figure 3b shows tested combinations as to the MS2 protein and the transcriptional activation domain (p65-HSF1). Average ratios between response in a photo state and response in a dark state for all the tested combinations are shown at a real number scale in Figure 3c and at a log2 scale in Figure 3d as a heat map. These ratios between response in a photo state and response in a dark state were calculated on the basis of the bioluminescence intensity of HEK 293T cells harboring CPTS and a bioluminescent reporter plasmid. Experimental conditions of Figures 3e and 3f were the same as in Figures 3c and 3d. A control using a C20A mutant or a C20S mutant of RpBphP1 exhibited high luminescence intensity equivalent to wild-type (WT) RpBphP1 under a red light condition in Figure 3e, whereas the luminescence completely disappeared in Figure 3f.

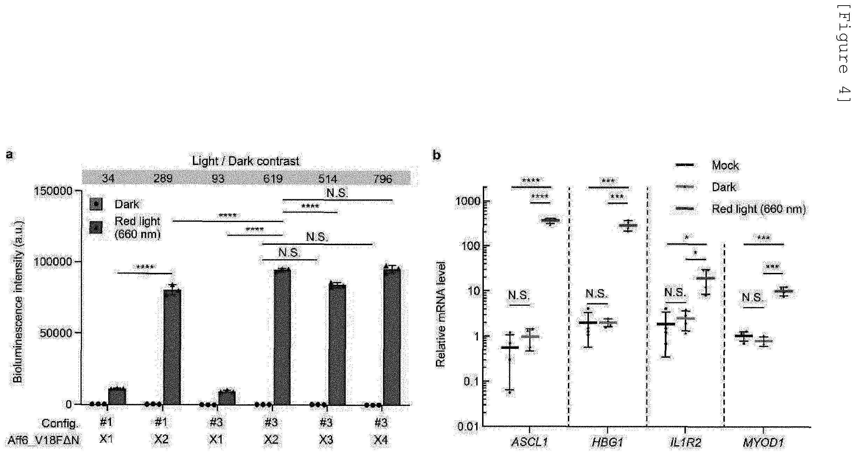

[Figure 4] Figure 4 shows results of optically manipulating gene expression in mammalian cells by CPTS using MagRed (Red-CPTS). Figure 4a shows results of optically manipulating gene expression by Red-CPTS using MagRed and CRISPR-Cas9. Red-CPTS was prepared with different designs and evaluated by using the bioluminescence intensity of HEK 293T cells as an index. The design (#1 and #3) of each configuration is shown in Figure 3. The drawing shows that the gene expression activity of Red-CPTS can be drastically enhanced by beading two or more molecules of Aff6_V18FΔN constituting MagRed. The right bar depicts response at the time of red light irradiation, and the left bar depicts response in the dark. The upper part of the drawing depicts the ratio (numeric value) between response at the time of red light irradiation and response in the dark. Figure 4b shows results of using a mixture of guide RNA molecules (gRNA) respectively targeting endogenous genes ASCL1, HBG1, IL1R2, and MYOD1 in HEK 293T cells to activate the genes at the same time with red light.

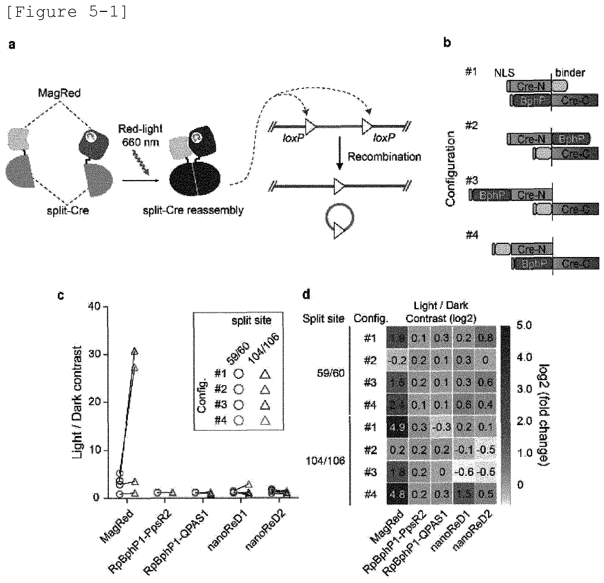

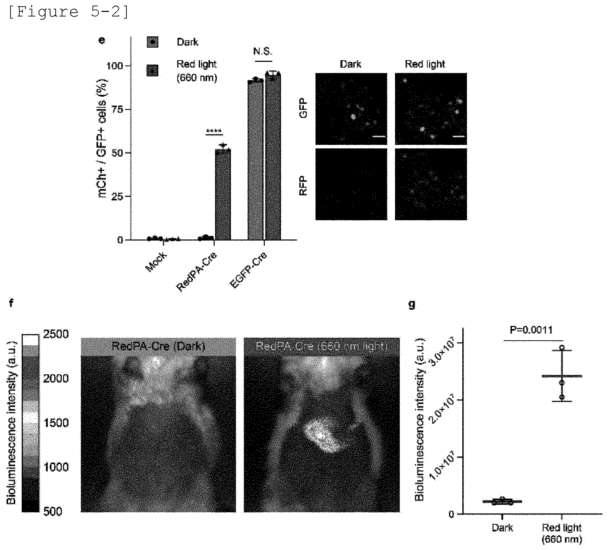

[Figure 5] Figure 5 shows a red light-driven DNA recombination system using MagRed. Figure 5a shows a schematic view of RedPA-Cre. Upon irradiation with light at 660 nm, both fragments of split Cre recombinase (split-Cre) bind to each other through the function of MagRed, leading to their activation so that the recombination reaction of DNA flanked by two loxP sequences occurs. Figure 5b shows four configurations as to the fusion of split Cre recombinase (split-Cre) with BphP and its binding partner. Their ratios between response in a photo state and response in a dark state were calculated on the basis of the bioluminescence intensity of HEK 293T cells harboring each of the designed molecules of RedPA-Cre and a bioluminescent reporter plasmid. Average ratios between response in a photo state and response in a dark state for all the tested combinations were plotted at a real number scale in Figure 5c and are shown at a log2 scale in Figure 5d as a heat map. These ratios between response in a photo state and response in a dark state were calculated on the basis of the bioluminescence intensity of HEK 293T cells harboring each of the four designed molecules of RedPA-Cre and a bioluminescent reporter plasmid. Figure 5e shows results of assay using a fluorescent reporter (mCherry, detected in an RFP channel under a fluorescence microscope) of RedPA-Cre linked to yellow fluorescent protein (Venus, detected in a GFP channel under a fluorescence microscope). The ratio (mCh+/GFP+) of mCherry-positive cells to Venus-positive cells was estimated from 1000 or more cells in three independent experiments. In Figure 5f, a bioluminescent reporter plasmid that could cause bioluminescence through DNA recombination reaction with a plasmid encoding RedPA-Cre was introduced to the liver of each mouse by hydrodynamic tail vein injection (HTV injection), and the mouse was subjected to noninvasive light irradiation with red LED ex *vivo.* The drawing shows results of comparing bioluminescence images of a mouse raised in the dark and the mouse irradiated with red light. The expression of luciferase from the bioluminescent reporter plasmid was observed by intraperitoneally introducing 200 µL of 100 mM D-luciferin to the mouse 25 hours after hydrodynamic tail vein injection, and imaging bioluminescence. Figure 5g shows results of comparing bioluminescence intensity between the mouse raised in the dark and the mouse irradiated with red light. The horizontal line with a bar depicts mean ± s.d. of bioluminescence intensity, and the dot depicts the bioluminescence intensity of each mouse.

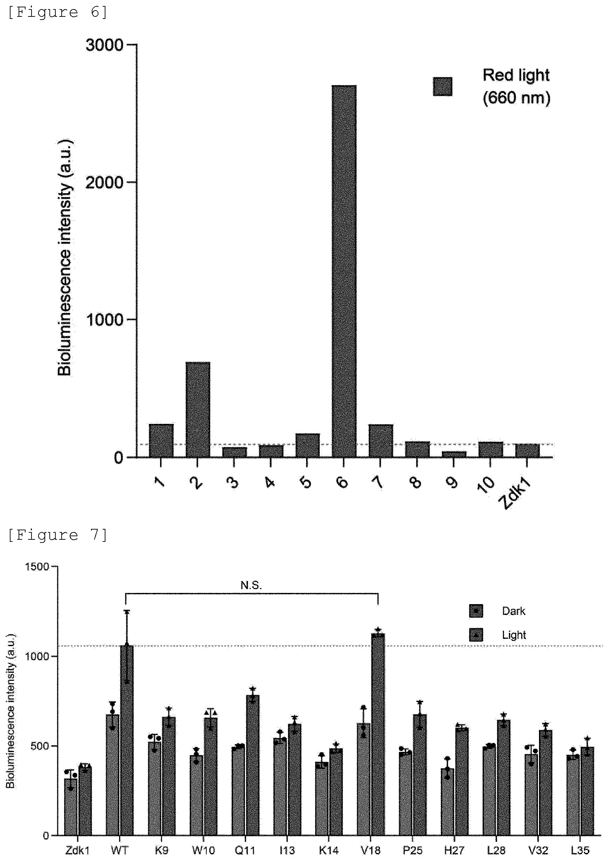

[Figure 6] Figure 6 shows results of analyzing ten types of Affibody molecules obtained by ribosome display screening. The nucleotide sequences (Table 1) of the ten types of Affibody molecules were introduced to upstream of the nucleotide sequence of VP16-NLS to prepare constructs. These constructs were introduced together with a construct of tetR-DrBphP-PSM and SEAP reporter to HeLa cells, and the ten types of Affibody molecules were compared by using bioluminescence intensity as an index. Zdk1 is Affibody (negative control) irrelevant to DrBphP-PSM. The analysis results were indicated in a mean of data obtained by three experiments.
[Figure 7] Figure 7 shows results of comparing the cases where amino acids at 12 residues among 13 randomizable residues of Affi6 were each substituted by alanine. In the drawing, the label depicts the amino acid residue substituted by alanine. The nucleotide sequence of Aff6 in a construct of Aff6-VP16-NLS was replaced with the nucleotide sequence of Aff6 having each substitution by alanine, and the resultant was introduced together with a construct of tetR-DrBphP and a bioluminescent reporter to HeLa cells. The alanine substitution variants were compared by using bioluminescence intensity as an index. The results were indicated in mean ± standard deviation of data obtained by three experiments. N.S. represents P > 0.05 (two-way ANOVA by multiple comparison).

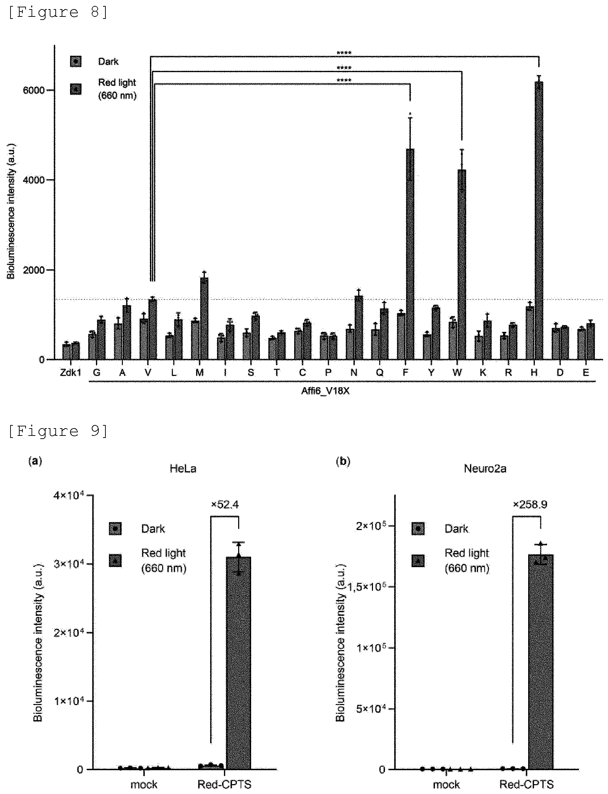

[Figure 8] Figure 8 shows results of comparing the cases where site-directed saturation mutation was introduced to an amino acid at position 18 (valine, V) of Aff6. In the drawing, the label depicts the amino acid (X) introduced at position 18 of an Aff6_V18X mutant. The nucleotide sequence of Aff6 in a construct of Aff6-VP16-NLS was replaced with the nucleotide sequence of Aff6_V18X, and the resultant was introduced together with a construct of tetR-DrBphP and a bioluminescent reporter to HeLa cells. The Aff6_V18X mutants were compared by using bioluminescence intensity as an index. The results were indicated in mean ± standard deviation of data obtained by three experiments. **** represents P < 0.0001 (two-way ANOVA by multiple comparison).
[Figure 9] Figure 9 shows the activation of Red-CPTS in various cell lines. A construct of Red-CPTS and a bioluminescent reporter was introduced to HeLa cells (a) or Neuro 2a cells (b), and the activation of Red-CPTS was evaluated by using bioluminescence intensity as an index. The ratio of average bioluminescence intensity between red light (right side of the bar graph) and dark (left side of the bar graph) conditions was indicated in numeric value. The results were indicated in a mean of data obtained by three experiments.

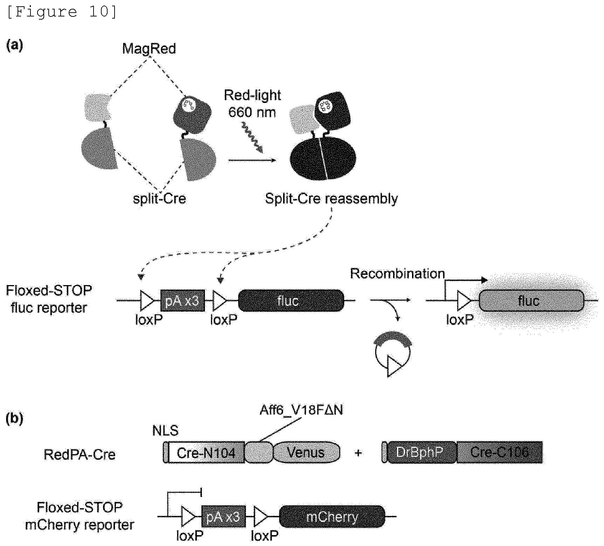

[Figure 10] Figure 10 shows results of evaluating RedPA-Cre by reporter assay. Figure 10a schematically shows reporter assay using RedPA-Cre. The expression of firefly luciferase (flue) is in a state suppressed by transcriptional stop signals (pA x3: polyadenylation transcriptional 'stop' signal tri-repeated sequence) located downstream of CMV promoter (this reporter is referred to as "Floxed-STOP flue reporter"). MagRed induces the rejoining of both fragments of split Cre recombinase (split-Cre) by light irradiation at 660 nm, and the transcriptional stop signal sequence flanked by two loxP sites is deleted through DNA recombination reaction mediated by the activated Cre recombinase so that the flue reporter is expressed through the function of the promoter. Figure 10b is a schematic view of reporter assay using RedPA-Cre linked to yellow fluorescent protein (Venus) (here, "Floxed-STOP mCherry reporter" is used in which red fluorescent protein mCherry is expressed through the function of RedPA-Cre).

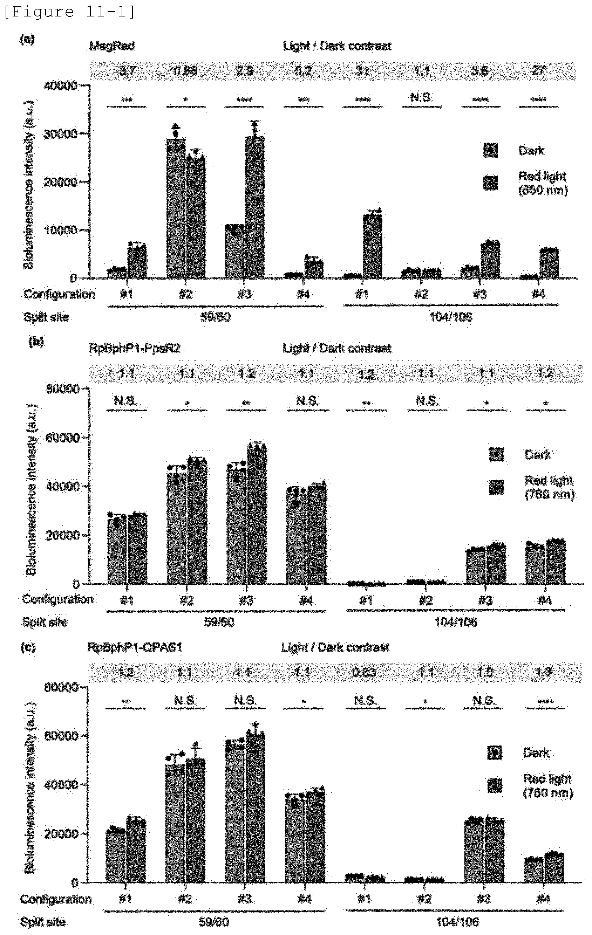

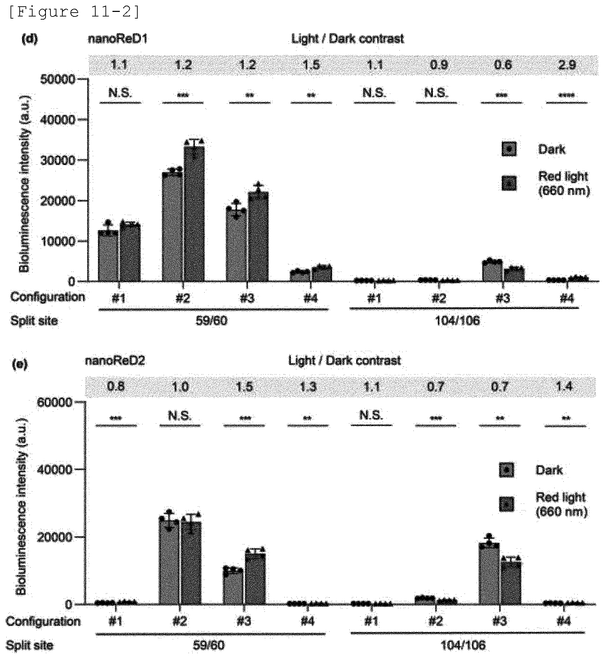

[Figure 11] Figure 11 shows results of evaluating the performance of each RedPA-Cre designed using five different red photoswitching proteins. Figures 11a to 11e show response at the time of red irradiation on the right side of the bar graph and show response in the dark on the left side of the bar graph. Floxed-STOP flue reporter was used in experiments. The design (#1-4) of each configuration is shown in Figure 5b. The upper part of the drawing depicts the ratio (numeric value) between response at the time of red light irradiation and response in the dark. N.S. represents P > 0.05; * represents P < 0.05; ** represents P < 0.01; *** represents P < 0.001; and **** represents P < 0.0001 (two-tailed unpaired t-test).
[Figure 12] Figure 12 shows results of evaluating the influence of biliverdin (BV) addition on the activity of RedPA-Cre. The evaluation was conducted by using bioluminescence intensity as an index in HEK 293T cells harboring RedPA-Cre and Floxed-STOP flue reporter. N.S. represents P > 0.05 (two-way ANOVA by multiple comparison).

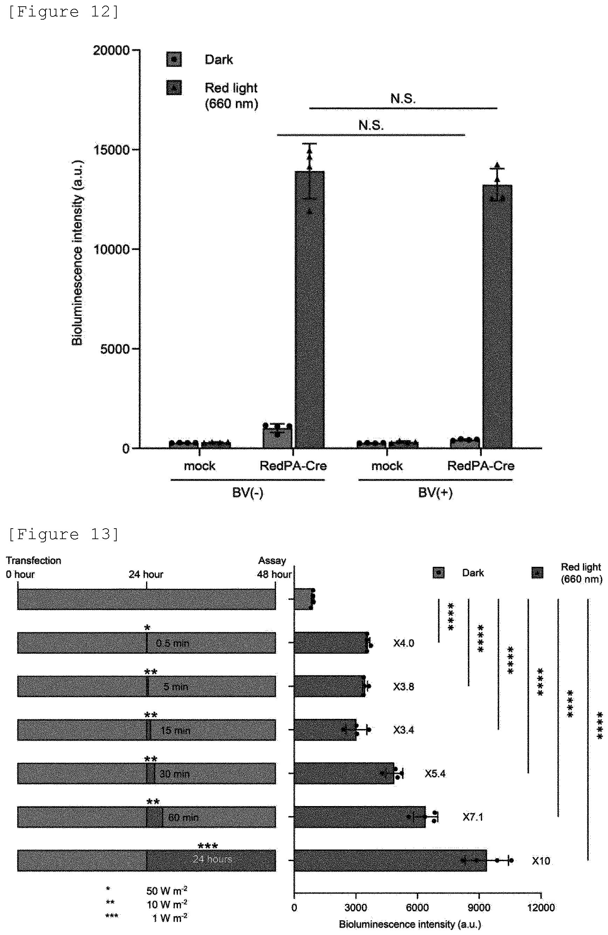

[Figure 13] Figure 13 shows evaluation results when RedPA-Cre was activated under various red light irradiation conditions. HEK 293T cells harboring RedPA-Cre and Floxed-STOP flue reporter were irradiated with red light using various times and intensities, and the DNA recombination activity of RedPA-Cre was evaluated. A region with the asterisk (*) corresponds to the time zone of red light irradiation at the designated intensity, and the other portions correspond to time zones in the dark. The ratio between each response at the time of red light irradiation and response in the dark is shown in the right of each bar graph. **** represents P < 0.0001 (one-way ANOVA by multiple comparison).

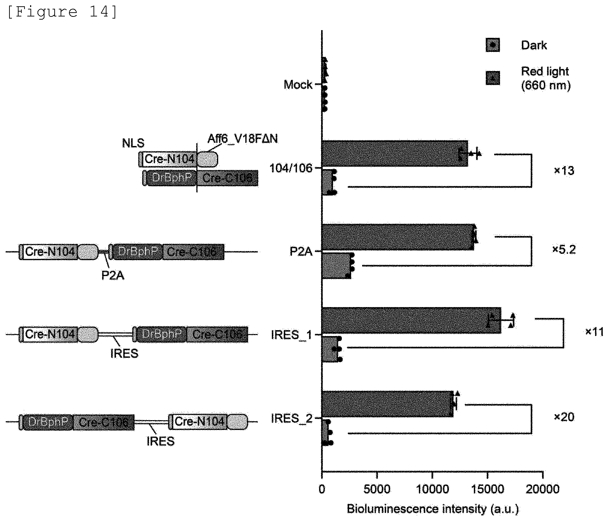

[Figure 14] Figure 14 shows results of introducing each of three bicistronic systems (P2A, IRES_1, and IRES_2) for expressing two different proteins by one vector to RedPA-Cre, followed by comparison by using bioluminescence intensity as an index. Response at the time of red light irradiation is shown on the upper side of the bar graph, and response in the dark is shown on the lower side of the bar graph. Floxed-STOP flue reporter was used in experiments. The ratio (numeric value) between response at the time of red light irradiation and response in the dark is shown on the right of the bar graph.

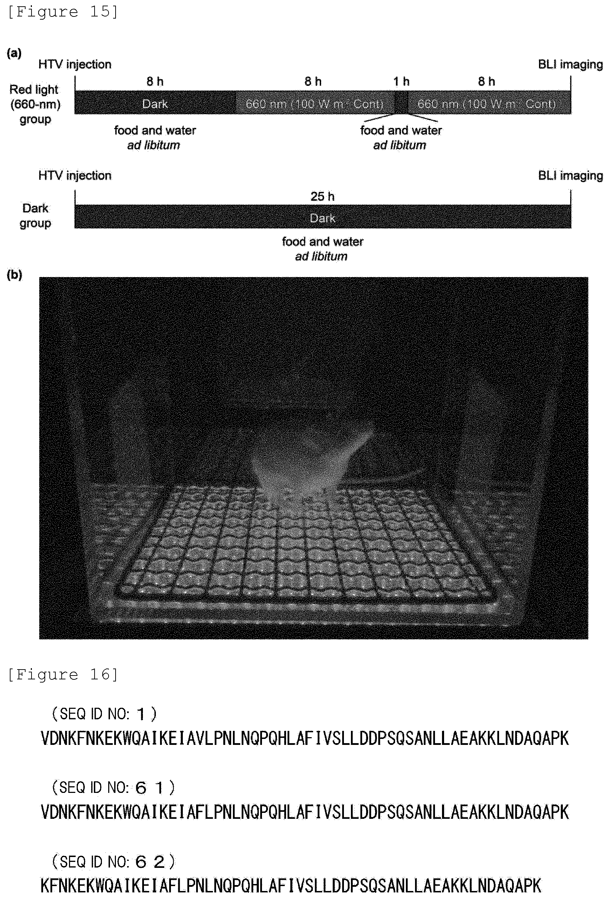

[Figure 15] Figure 15 shows results of experiments using RedPA-Cre in mice *in vivo.* In Figure 15a, experiments were conducted in order to evaluate the activation of RedPA-Cre in the mouse liver. RedPA-Cre containing IRES_2 and Floxed-STOP flue reporter were introduced (50 mg in total) at a plasmid ratio of 1:4 to each ICR mouse, which was then irradiated with red light at 100 W m-2 for 16 hours or raised in the dark. In Figure 15b, the mouse was subjected to noninvasive red light irradiation ex *vivo* using a red LED light source. The light irradiation of the mouse was performed in a transparent acrylic box (15 cm × 15 cm) wired at the bottom.
[Figure 16] Figure 16 shows the amino acid sequence of SEQ ID NO: 1 and also shows the amino acid sequences of SEQ ID NOs: 61 and 62. SEQ ID NOs: 1, 61, and 62 correspond to the amino acid sequences of the Affibody molecules designated as Aff6, Aff6_V18F, and Aff6_V18FΔN, respectively, in Figure 1.

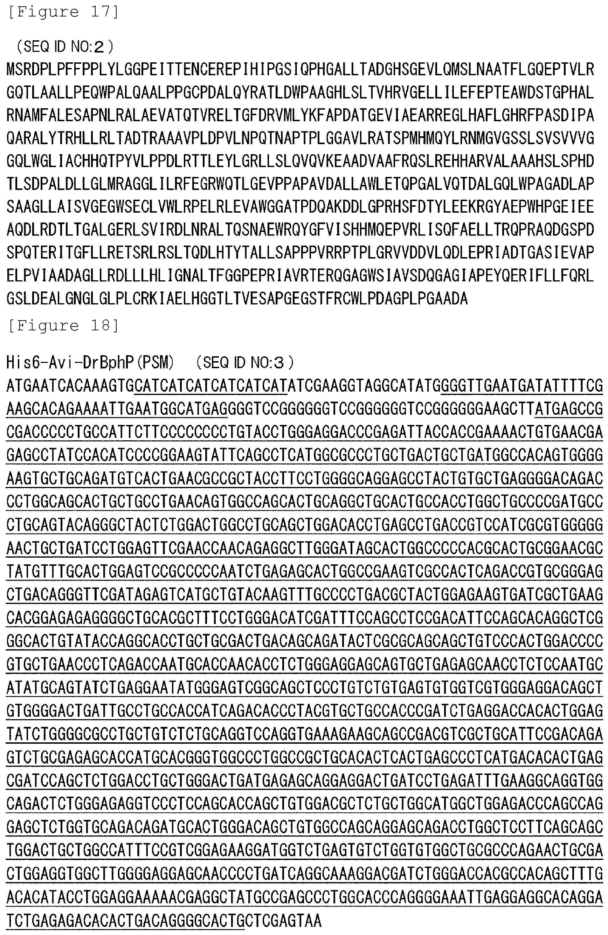

[Figure 17] Figure 17 shows the amino acid sequence of SEQ ID NO: 2. SEQ ID NO: 2 corresponds to the amino acid sequence of the photoreceptor protein designated as DrBphP in Figure 1.
[Figure 18] Figure 18 shows a DNA sequence (SEQ ID NO: 3) used in synthesizing His6-Avi-DrBphP(PSM). A portion related to each domain is underlined in each sequence.

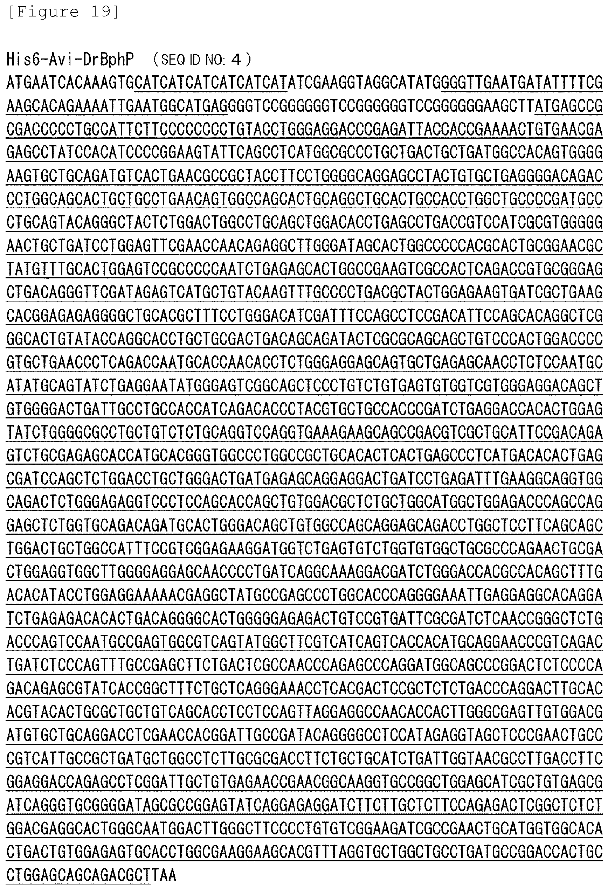

[Figure 19] Figure 19 shows a DNA sequence (SEQ ID NO: 4) used in synthesizing His6-Avi-DrBphP.

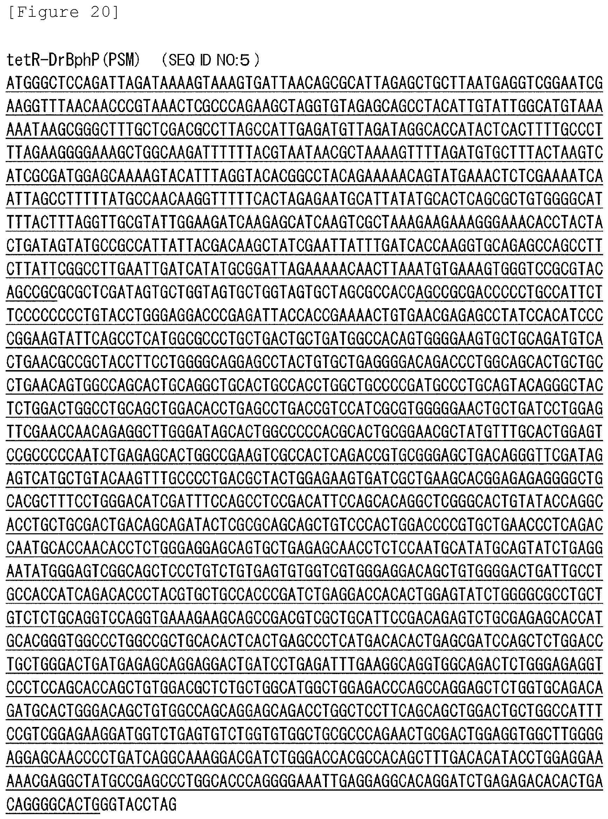

[Figure 20] Figure 20 shows a DNA sequence (SEQ ID NO: 5) used in synthesizing tetR-DrBphP(PSM).

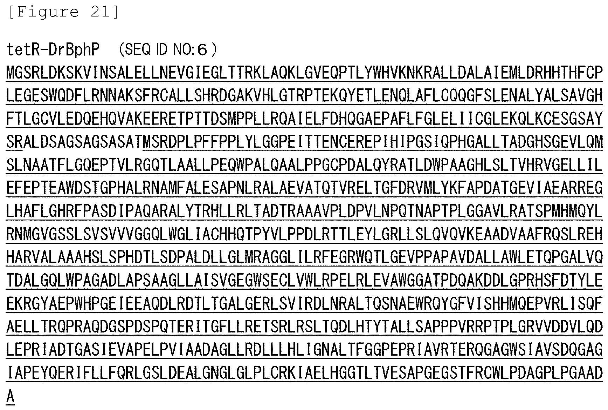

[Figure 21] Figure 21 shows the amino acid sequence (SEQ ID NO: 6) of tetR-DrBphP.

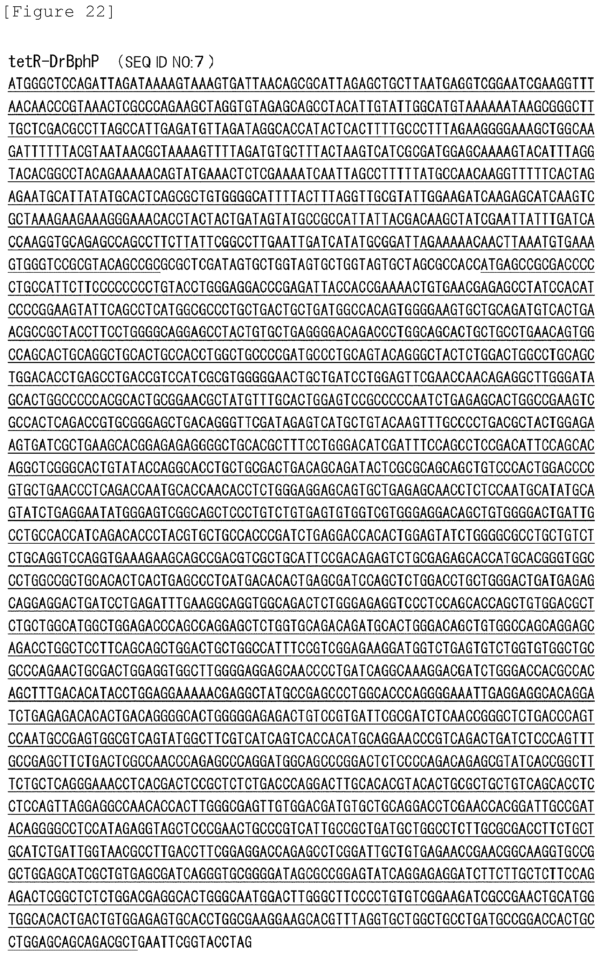

[Figure 22] Figure 22 shows a DNA sequence (SEQ ID NO: 7) used in synthesizing tetR-DrBphP.

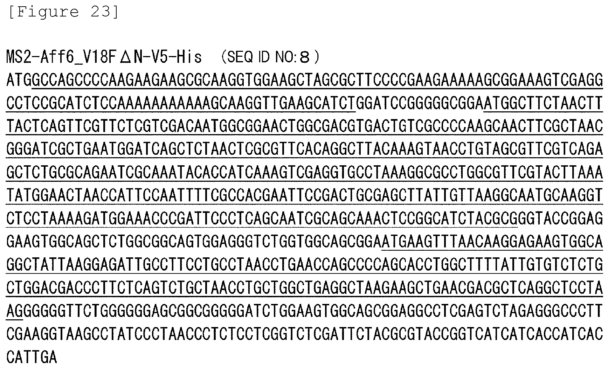

[Figure 23] Figure 23 shows a DNA sequence (SEQ ID NO: 8) used in synthesizing 3xNLS-MS2-DrBphP-V5-His.

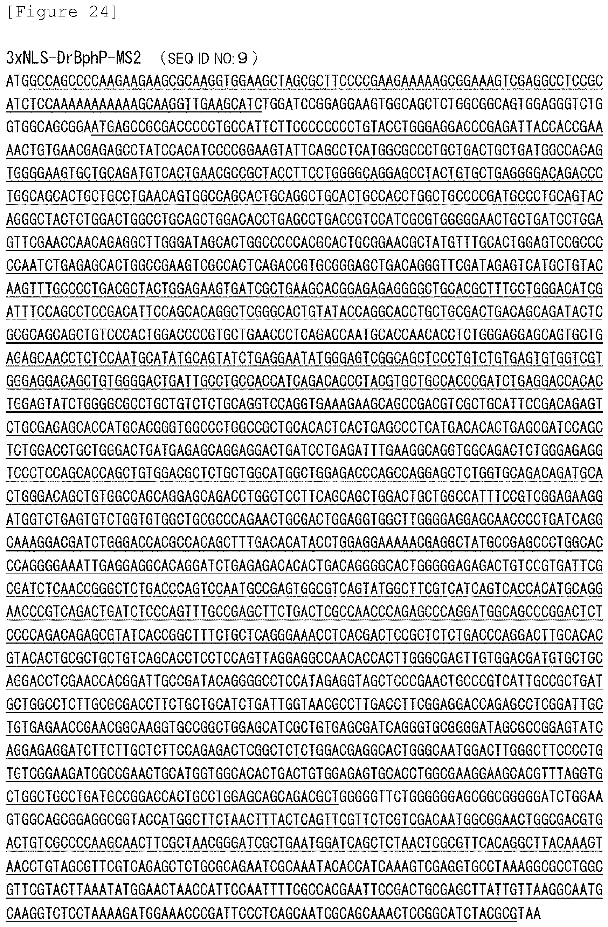

[Figure 24] Figure 24 shows a DNA sequence (SEQ ID NO: 9) used in synthesizing 3xNLS-DrBphP-MS2.

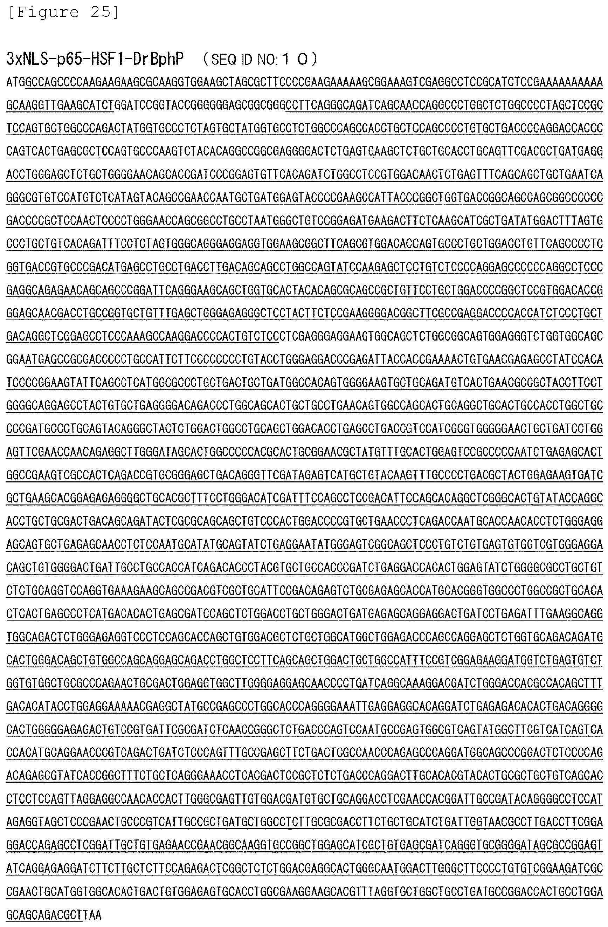

[Figure 25] Figure 25 shows a DNA sequence (SEQ ID NO: 10) used in synthesizing 3xNLS-p65-HSF1-DrBphP.

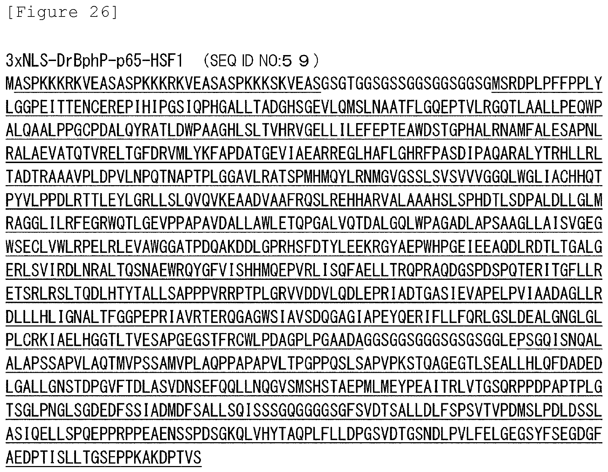

[Figure 26] Figure 26 shows the amino acid sequence (SEQ ID NO: 59) of 3xNLS-DrBphP-p65-HSF1.

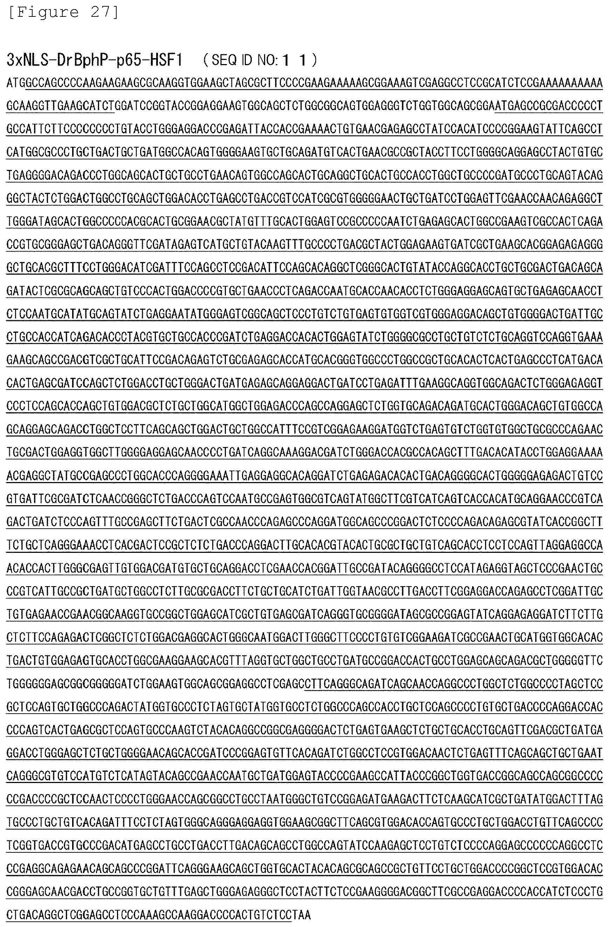

[Figure 27] Figure 27 shows a DNA sequence (SEQ ID NO: 11) used in synthesizing 3xNLS-DrBphP-p65-HSF1.

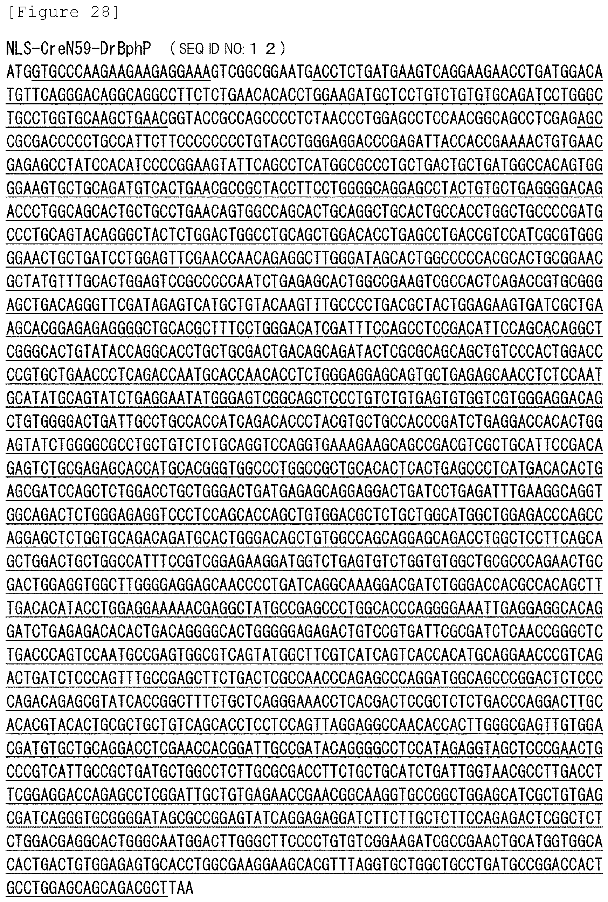

[Figure 28] Figure 28 shows a DNA sequence (SEQ ID NO: 12) used in synthesizing NLS-CreN59-DrBphP.

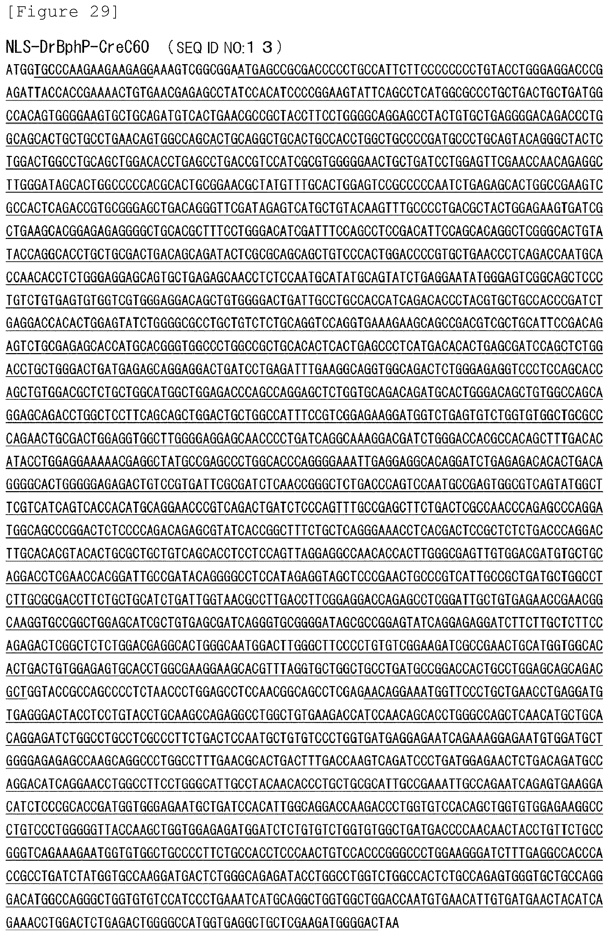

[Figure 29] Figure 29 shows a DNA sequence (SEQ ID NO: 13) used in synthesizing NLS-DrBphP-CreC60.

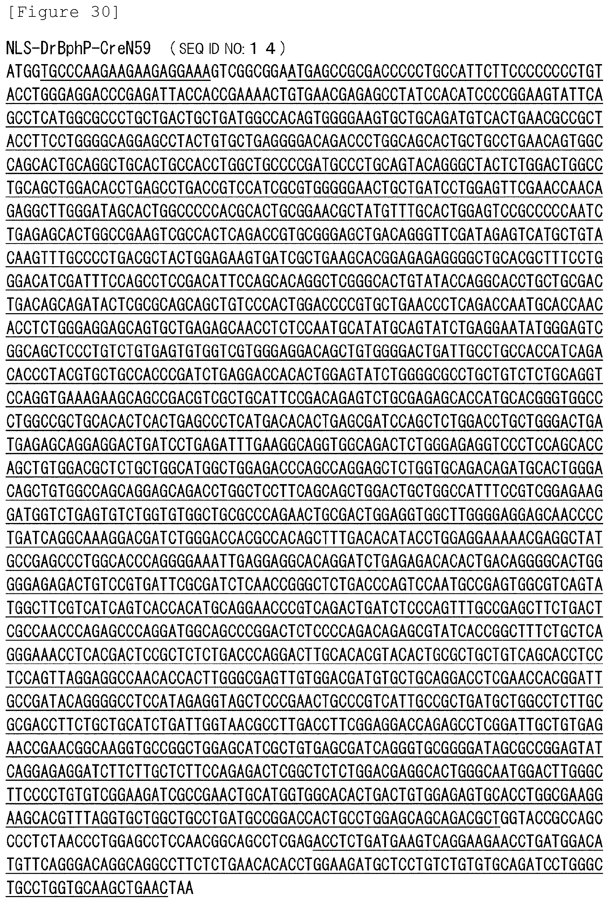

[Figure 30] Figure 30 shows a DNA sequence (SEQ ID NO: 14) used in synthesizing NLS-DrBphP-CreN59.

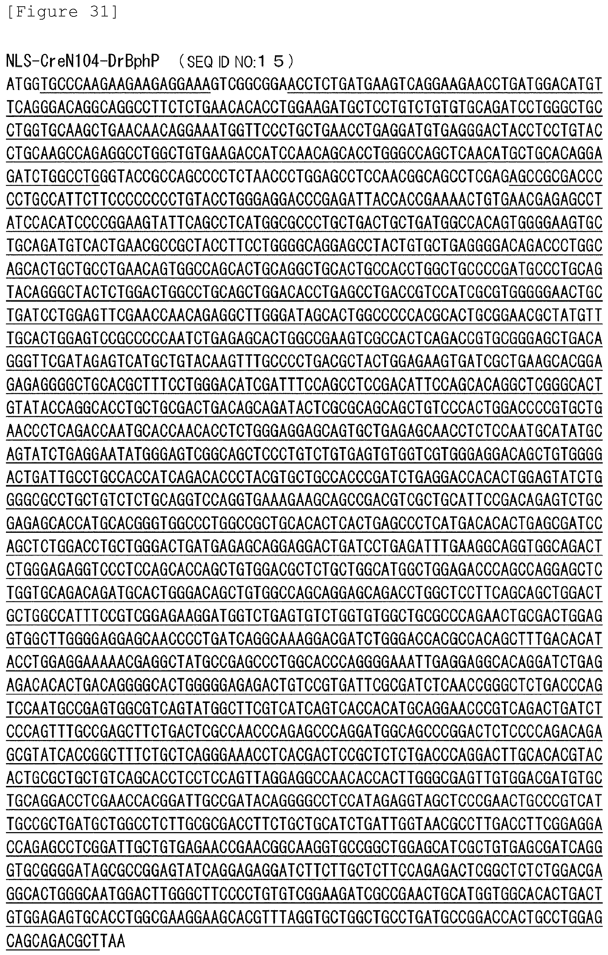

[Figure 31] Figure 31 shows a DNA sequence (SEQ ID NO: 15) used in synthesizing NLS-CreN104-DrBphP.

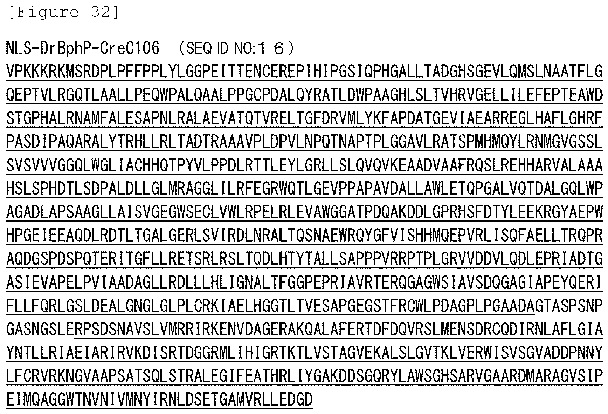

[Figure 32] Figure 32 shows the amino acid sequence (SEQ ID NO: 16) of NLS-DrBphP-CreC106.

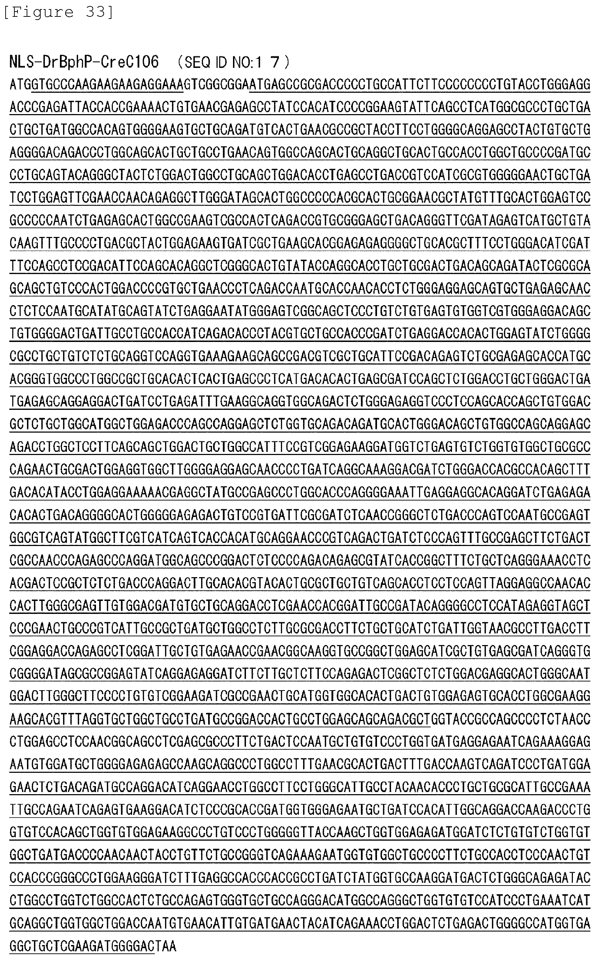

[Figure 33] Figure 33 shows a DNA sequence (SEQ ID NO: 17) used in synthesizing NLS-DrBphP-CreC106.

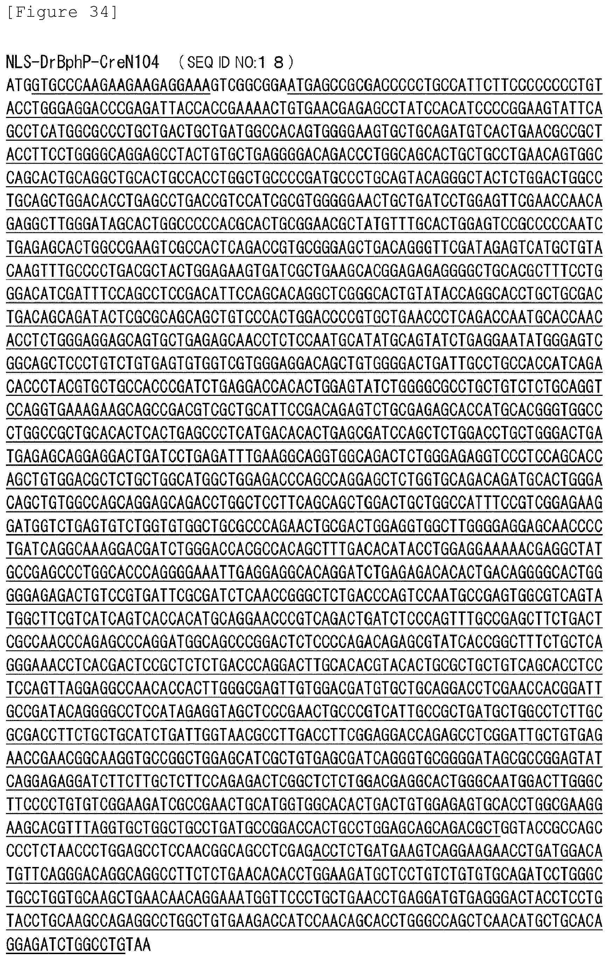

[Figure 34] Figure 34 shows a DNA sequence (SEQ ID NO: 18) used in synthesizing NLS-DrBphP-CreN104.

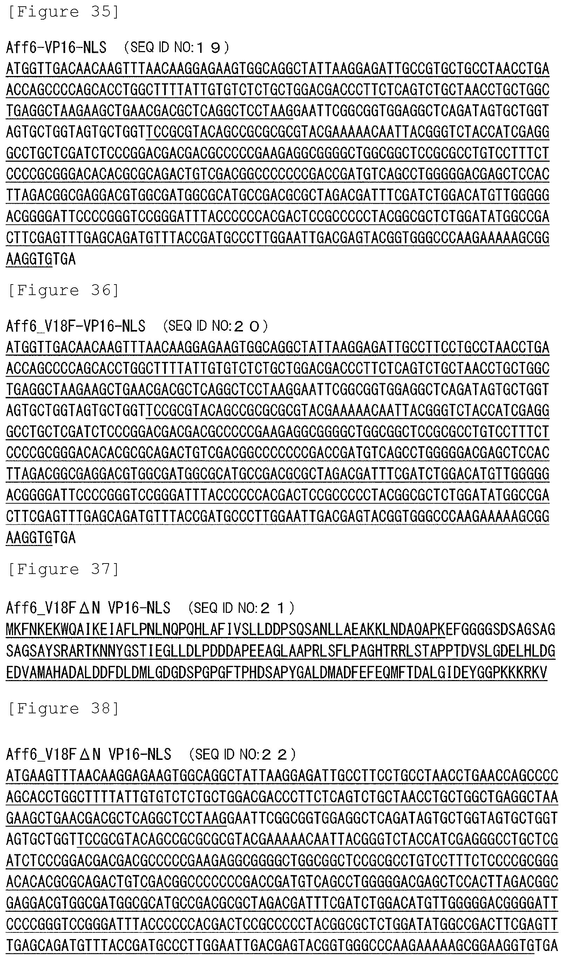

[Figure 35] Figure 30 shows a DNA sequence (SEQ ID NO: 19) used in synthesizing Aff6-VP16-NLS.
[Figure 36] Figure 36 shows a DNA sequence (SEQ ID NO: 20) used in synthesizing Aff6_V18F-VP16-NLS.
[Figure 37] Figure 37 shows the amino acid sequence (SEQ ID NO: 21) of Aff6_V18FΔN VP16-NLS.
[Figure 38] Figure 38 shows a DNA sequence (SEQ ID NO: 22) used in synthesizing Aff6_V18FΔN VP16-NLS.

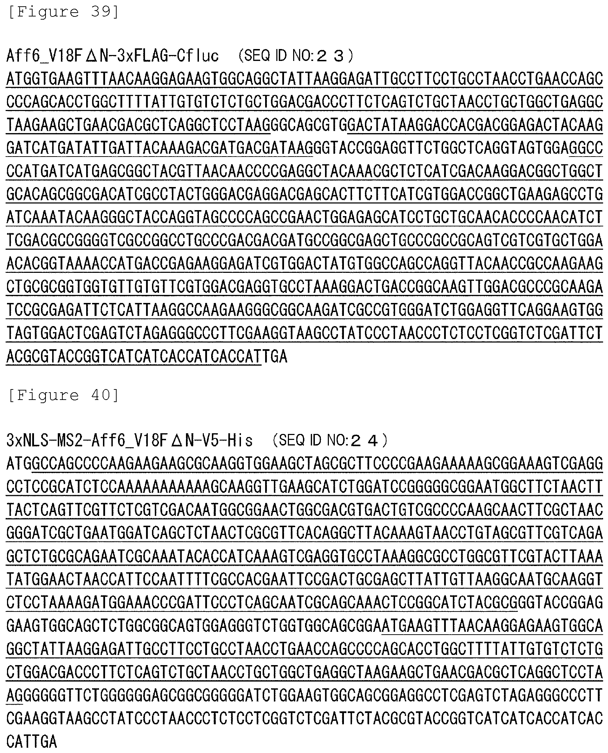

[Figure 39] Figure 39 shows a DNA sequence (SEQ ID NO: 23) used in synthesizing Aff6_V18FΔN-3xFLAG-Cfluc.
[Figure 40] Figure 40 shows a DNA sequence (SEQ ID NO: 24) used in synthesizing 3xNLS-MS2-Aff6_V18FΔN-V5-His.

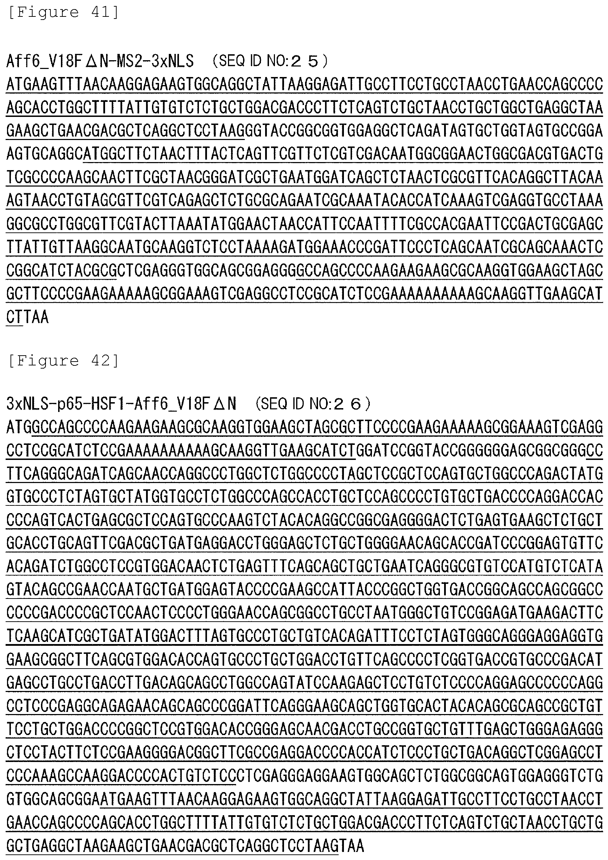

[Figure 41] Figure 41 shows a DNA sequence (SEQ ID NO: 25) used in synthesizing Aff6_V18FΔN-MS2-3xNLS.
[Figure 42] Figure 42 shows a DNA sequence (SEQ ID NO: 26) used in synthesizing 3xNLS-p65-HSF1-Aff6_V18FΔN.

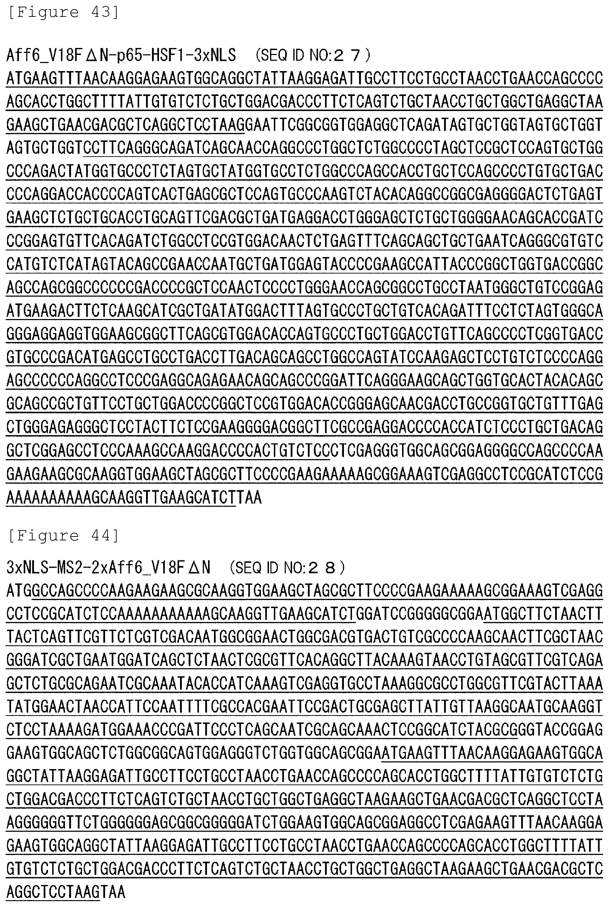

[Figure 43] Figure 43 shows a DNA sequence (SEQ ID NO: 27) used in synthesizing Aff6_V18FΔN-p65-HSF1-3xNLS.
[Figure 44] Figure 44 shows a DNA sequence (SEQ ID NO: 28) used in synthesizing 3xNLS-MS2-2xAff6_V18FΔN.

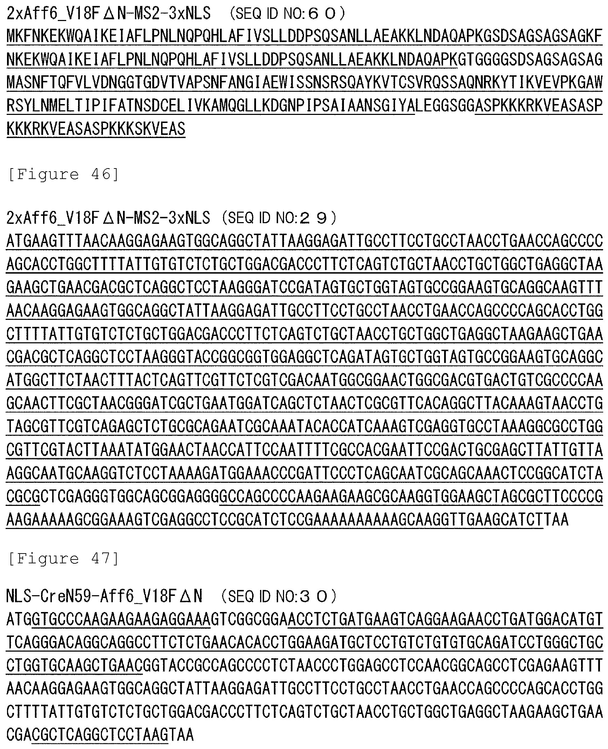

[Figure 45] Figure 45 shows the amino acid sequence (SEQ ID NO: 60) of 2xAff6_V18FΔN-MS2-3xNLS.
[Figure 46] Figure 46 shows a DNA sequence (SEQ ID NO: 29) used in synthesizing 2xAff6_V18FΔN-MS2-3xNLS.
[Figure 47] Figure 47 shows a DNA sequence (SEQ ID NO: 30) used in synthesizing NLS-CreN59-Aff6_V18FΔN.

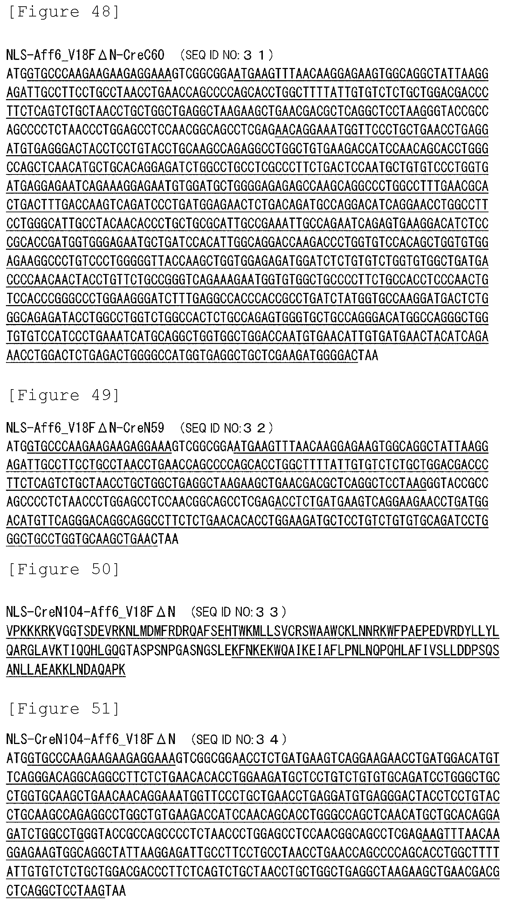

[Figure 48] Figure 48 shows a DNA sequence (SEQ ID NO: 31) used in synthesizing NLS-Aff6_V18FΔN-CreC60.
[Figure 49] Figure 49 shows a DNA sequence (SEQ ID NO: 32) used in synthesizing NLS-Aff6_V18FΔN-CreN59.
[Figure 50] Figure 50 shows the amino acid sequence (SEQ ID NO: 33) of NLS-CreN104-Aff6_V18FΔN.
[Figure 51] Figure 51 shows a DNA sequence (SEQ ID NO: 34) used in synthesizing NLS-CreN104-Aff6_V18FΔN.

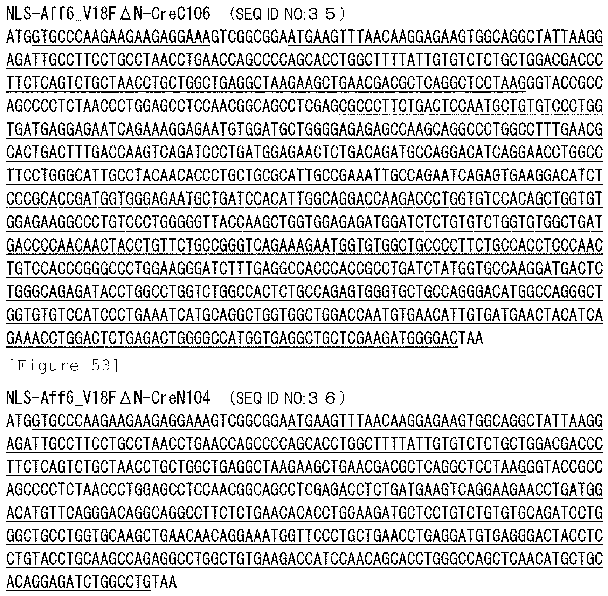

[Figure 52] Figure 52 shows a DNA sequence (SEQ ID NO: 35) used in synthesizing NLS-Aff6_V18FΔN-CreC106.
[Figure 53] Figure 53 shows a DNA sequence (SEQ ID NO: 36) used in synthesizing NLS-Aff_ V18FΔN-CreN104.

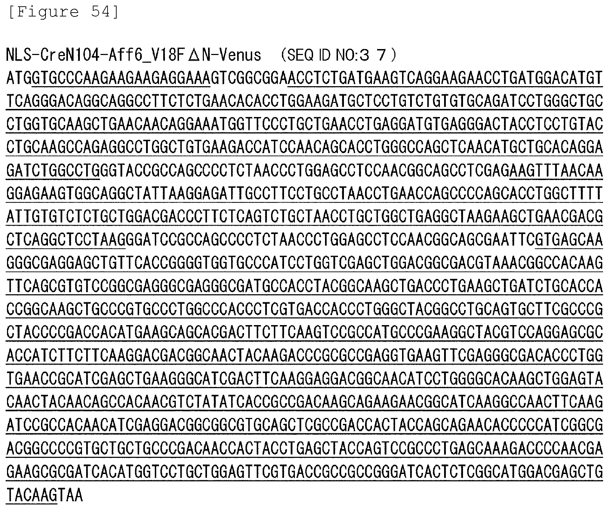

[Figure 54] Figure 54 shows a DNA sequence (SEQ ID NO: 37) used in synthesizing NLS-CreN104-Aff6_V18FΔN-Venus.

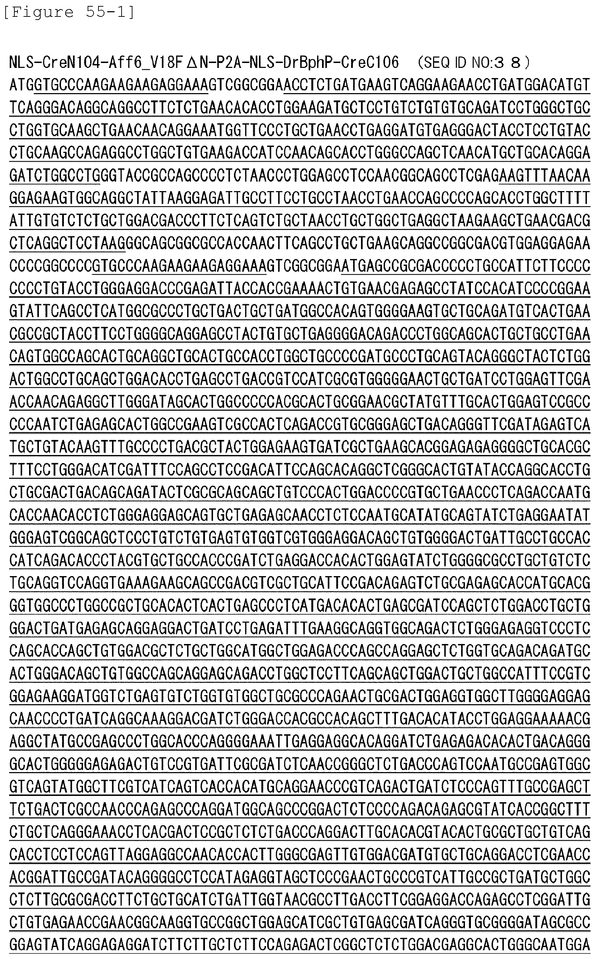

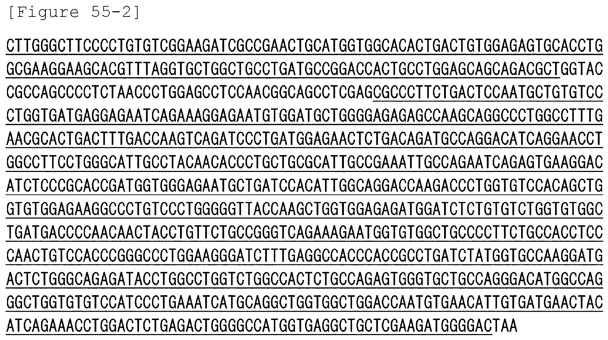

[Figure 55] Figure 55 shows a DNA sequence (SEQ ID NO: 38) used in synthesizing NLS-CreN104-Aff6_V18FΔN-P2A-NLS-DrBphP-CreC106.

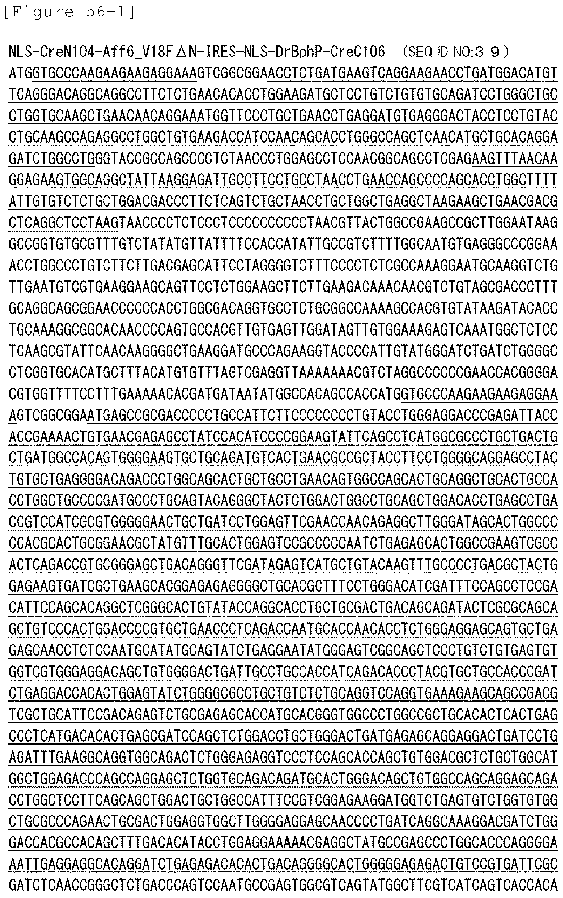

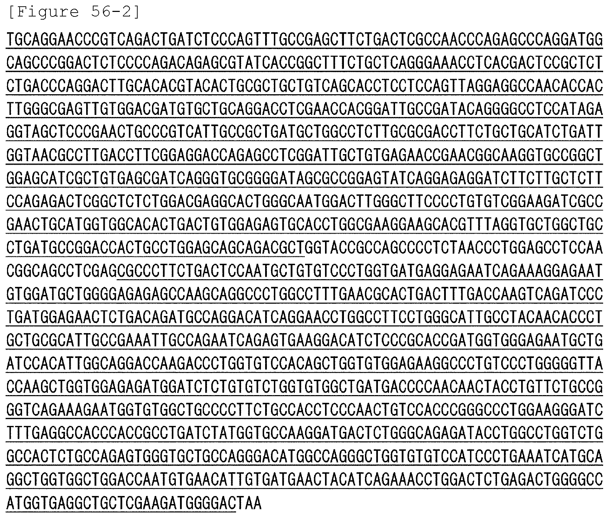

[Figure 56] Figure 56 shows a DNA sequence (SEQ ID NO: 39) used in synthesizing NLS-CreN104-Aff6_V18FΔN-IRES-NLS-DrBphP-CreC106.

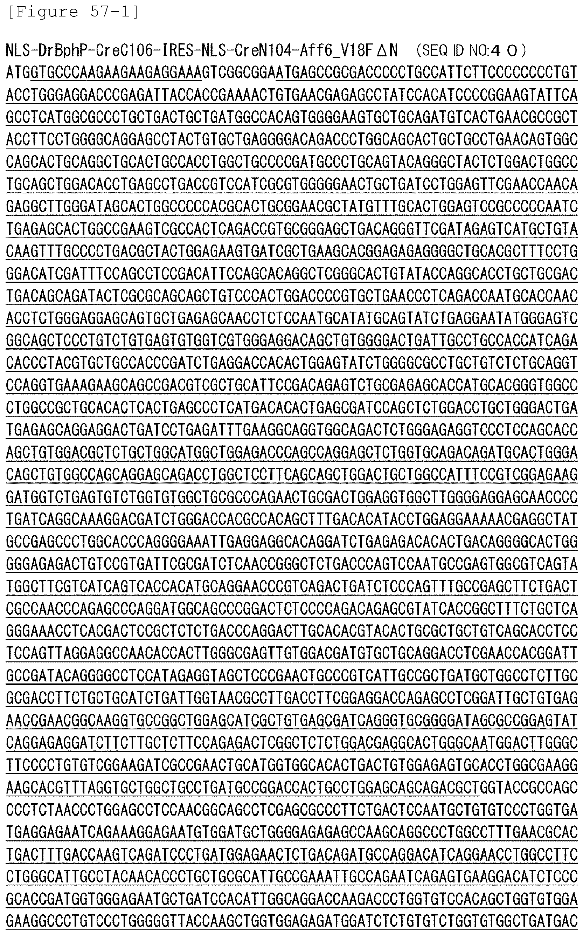

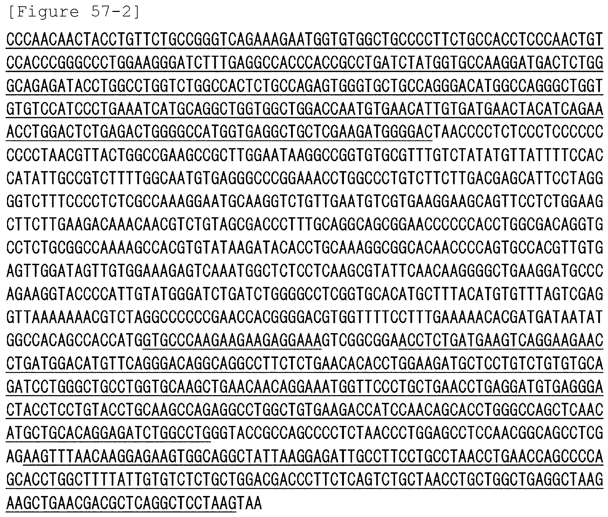

[Figure 57] Figure 57 shows a DNA sequence (SEQ ID NO: 40) used in synthesizing NLS-DrBphP-CreC106-IRES-NLS-CreN104-Aff6_V18FΔN.

### Description of Embodiments

Hereinafter, the mode for carrying out the present invention will be described in detail. The present invention is not limited to the following embodiments.

The set of Affibody and photoreceptor protein of the present invention is a set wherein the Affibody and the photoreceptor protein form a complex in a manner dependent on light at a wavelength of 600 to 750 nm.

The photoreceptor protein used in the present invention is a prokaryotic photoreceptor protein and is known as bacteriophytochrome. In the present invention, the photoreceptor protein is a protein also abbreviated to BphP.

The photoreceptor protein is known to vary in structure depending on light in different long wavelength regions.

The photoreceptor protein is not particularly limited as long as the Affibody binds thereto by red light and dissociates therefrom by near-infrared light.

Examples of the photoreceptor protein include, but are not particularly limited to, DrBphP known as bacterium (*Deinococcus radiodurans*)*-derived* red sensor protein.

DrBphP has an N-terminal photosensory module (PSM). DrBphP shifts its state from Pr (dark state) to Pfr (photo state) upon irradiation with red light. DrBphP in the photo state binds to the Affibody.

The photoreceptor protein will be described by taking DrBphP as an example. DrBphP binds with an endogenous dye as a cofactor and DrBphP does not require further addition of the cofactor for forming a complex with the Affibody because its intracellular concentration satisfies a requirement for the concentration of the cofactor. In addition, the complex formation using the set of Affibody and photoreceptor protein of the present invention has the advantage that specific binding occurs in the presence of red light while the complex is not formed in the dark. As also disclosed in Examples, the set of Affibody and photoreceptor protein of the present invention is capable of controlling the association of a split protein by ON/OFF of red light.

The photoreceptor protein as DrBphP preferably
(1) has the amino acid sequence represented by SEQ ID NO: 2, or
(2) has an amino acid sequence having 80% or higher homology to the amino acid sequence represented by SEQ ID NO: 2.

As used herein, the phrase "having 80% or higher homology to the amino acid sequence represented by SEQ ID NO" means that a mutation is acceptable for the particular SEQ ID NO.

In this context, the "80% or higher homology" means that amino acids overlapping with the amino acid sequence represented by the particular SEQ ID NO account for at least 80% of the total number of amino acids in the amino acid sequence represented by this SEQ ID NO.

For example, when the amino acid sequence represented by SEQ ID NO consists of 100 amino acids, the 80% or higher homology means that the sequence having this homology may consist of 80 amino acids including 80 homologous amino acids (homology = 80%), or means that the sequence having this homology may consist of 120 amino acids including 100 homologous amino acids (homology = 100%). Alternatively, the term means that the sequence having this homology may consist of 100 amino acids including 80 homologous amino acids (homology = 80%).

In the present specification, in the case of "having 80% or higher homology to the amino acid sequence represented by SEQ ID NO", the homology is preferably 85% or higher, 90% or higher, 95% or higher, 98% or higher, or 100%.

Since the case of "having 80% or higher homology to the amino acid sequence represented by SEQ ID NO" includes the case of having 100% homology, the sequence having this homology may be the "amino acid sequence represented by SEQ ID NO: 2" unless otherwise specified.

In the case of "having 80% or higher homology to the amino acid sequence represented by SEQ ID NO", one to several amino acids may be added, substituted, or deleted in the amino acid sequence represented by SEQ ID NO.

In this context, the term "several" can be 10 or less, 9 or less, 8 or less, 7 or less, 6 or less, 5 or less, 4 or less, 3 or less, or 2 or less. Specifically, 1, 1 to 2, 1 to 3, 1 to 4, 1 to 5, 1 to 6, 1 to 7, 1 to 8, 1 to 9, or 1 to 10 amino acids may be added, substituted, or deleted in the amino acid sequence represented by SEQ ID NO.

The upper limit of the number of amino acids that may be added, substituted, or deleted in the amino acid sequence represented by SEQ ID NO can be determined within the range of the homology as long as the homology is 80% or higher, 85% or higher, 90% or higher, 95% or higher, or 98% or higher.

In the present specification, the case of containing addition, substitution, or deletion in a certain amino acid sequence means that the addition (insertion), the substitution, or the deletion occurs at an end or a site other than the end in the sequence. The number of amino acids to be added, substituted, or deleted is not particularly limited as long as the resulting polypeptide exerts the effects according to the present invention. The number of sites at which addition, substitution, or deletion occurs may be one or may be two or more.

In the present specification, the homology to the amino acid sequence represented by SEQ ID NO can be calculated by aligning the sequences by a method known to those skilled in the art so as to attain the highest match of amino acids therebetween, and determining the ratio of the amino acids overlapping with the amino acid sequence represented by SEQ ID NO.

In the present specification, the homology may be interchanged with identity.

When the photoreceptor protein is DrBphP, it is preferred that cysteine at position 24 counted from the N terminus in SEQ ID NO: 1 not be mutated because this amino acid serves as a covalent binding site for a cofactor biliverdin and therefore binds to biliverdin.

DrBphP is known to be composed mainly of four domains (PAS domain, GAF domain, PHY domain, and HK domain). The PAS domain, the GAF domain, and the PHY domain have a high commonality in proteins generically named as "bacteriophytochrome". On the other hand, the HK domain, also called effector domain, is known to often differ depending on the type of the bacteriophytochrome. A mutation may be provided in each domain on the basis of such information.

The Affibody used in the present invention means a category of proteins derived from a protein consisting of approximately 58 amino acids designed on the basis of the Z domain of *Staphylococcus aureus* protein A. The Affibody has a variable region regarded as a binding site for the photoreceptor protein, and a constant region used for maintaining the structure of the Affibody.

The Affibody used in the present invention, typically, can be
(1) a peptide having the amino acid sequence represented by SEQ ID NO: 1.

In SEQ ID NO: 1, amino acids at positions 9 to 11, 13, 14, 17, 18, 24, 25, 27, 28, 32, and 35 reside on the Affibody surface in the helix bundle of the Affibody and are involved in binding to the photoreceptor protein. Specifically, in SEQ ID NO: 1, the amino acids at positions 9 to 11, 13, 14, 17, 18, 24, 25, 27, 28, 32, and 35 constitute the variable region of the Affibody.

The amino acids of the Affibody involved in binding to the photoreceptor protein are amino acids considered to be involved in binding to the photoreceptor protein in a state capable of binding to the Affibody by light excitation, and are amino acids at positions 9 to 11, 13, 14, 17, 18, 24, 25, 27, 28, 32, and 35 in the amino acid sequence represented by SEQ ID NO: 1.

These amino acids are preferably conserved in SEQ ID NO: 1. However, even Affibody in which one or some of the amino acids are substituted by other amino acids has the ability to form a complex with the photoreceptor protein.

Specifically, the amino acids at positions 9 to 11, 13, 14, 17, 18, 24, 25, 27, 28, 32, and 35 in the amino acid sequence represented by SEQ ID NO: 1 may be mutated to other amino acids as long as the resulting protein has the ability to form a complex with the photoreceptor protein.

Amino acids other than the amino acids at positions 9 to 11, 13, 14, 17, 18, 24, 25, 27, 28, 32, and 35 in the amino acid sequence represented by SEQ ID NO: 1, i.e., amino acids at positions 1 to 8, 12, 15, 16, 19 to 23, 26, 29 to 31, 33, 34, and 36 to 58, are regarded as amino acids of the constant region in the Affibody.

The amino acids other than the amino acids at positions 9 to 11, 13, 14, 17, 18, 24, 25, 27, 28, 32, and 35 are amino acids that may be considered amino acids of the constant region, and are amino acids used for maintaining the structure of the Affibody. Accordingly, the amino acids at positions other than the positions 9 to 11, 13, 14, 17, 18, 24, 25, 27, 28, 32, and 35 in SEQ ID NO: 1 may be substituted or deleted, or other amino acids may be added thereto, as long as the amino acids at positions 9 to 11, 13, 14, 17, 18, 24, 25, 27, 28, 32, and 35 are a combination of amino acids having the ability to bind to the photoreceptor protein and as long as the structure of the Affibody is maintained.

Examples of the deletion in the Affibody include deletion of an N-terminal or a C-terminal amino acid of the Affibody.

The deletion of an N-terminal or a C-terminal amino acid may be deletion of 1, 2, 3, 4, 5, 6, 7, 8, 9, or 10 consecutive or nonconsecutive amino acids.

For example, 1 to 5 N-terminal or C-terminal amino acids may be deleted, 1 to 4 N-terminal or C-terminal amino acids may be deleted, 1 to 3 N-terminal or C-terminal amino acids may be deleted, 1 to 2 N-terminal or C-terminal amino acids may be deleted, or 1 N-terminal or C-terminal amino acid may be deleted. The deletion of these amino acids can be deletion of consecutive amino acids from each terminal amino acid.

Among the amino acids at positions other than the positions 9 to 11, 13, 14, 17, 18, 24, 25, 27, 28, 32, and 35 in SEQ ID NO: 1, 1, 2, 3, 4, 5, 6, 7, 8, 9, or 10 amino acids may be substituted.

1, 2, 3, 4, 5, 6, 7, 8, 9, or 10 amino acids may be added thereto as long as the amino acids at positions 9 to 11, 13, 14, 17, 18, 24, 25, 27, 28, 32, and 35 in SEQ ID NO: 1 have the ability to bind to the photoreceptor protein.

The number of amino acids to be substituted, added, or deleted in the amino acid sequence represented by SEQ ID NO: 1 in the Affibody can be 1 to 10, 1 to 9, 1 to 8, 1 to 7, or 1 to 6, and the upper limit may be 5 or less, 4 or less, 3 or less, or 2 or less or may be 1.

In particular, any amino acid at positions 12, 15, 16, 19 to 23, 26, 29 to 31, 33, and 34 among the amino acids located at positions 9 to 35 may be deleted as long as the amino acids at positions 9 to 11, 13, 14, 17, 18, 24, 25, 27, 28, 32, and 35 are capable of assuming a structure having the ability to bind to the photoreceptor protein.

The Affibody used in the present invention may have
(2) an amino acid sequence differing from the amino acid sequence represented by SEQ ID NO: 1 by mutation of an amino acid at one to five positions among positions 9 to 11, 13, 14, 17, 18, 24, 25, 27, 28, 32, and 35,
(3) an amino acid sequence having 80% or higher homology to the portions of the amino acid sequence represented by SEQ ID NO: 1, outside of the positions 9 to 11, 13, 14, 17, 18, 24, 25, 27, 28, 32, and 35,
(4) an amino acid sequence derived from the amino acid sequence represented by SEQ ID NO: 1, having mutation of an amino acid at one to five positions among positions 9 to 11, 13, 14, 17, 18, 24, 25, 27, 28, 32, and 35 and having 80% or higher homology to the portions of the amino acid sequence represented by SEQ ID NO: 1, outside the positions 9 to 11, 13, 14, 17, 18, 24, 25, 27, 28, 32, and 35,
(5) an amino acid sequence differing from the amino acid sequence represented by SEQ ID NO: 1 by mutation of an amino acid at position 18 and/or 24,
(6) an amino acid sequence derived from the amino acid sequence represented by SEQ ID NO: 1, having mutation of an amino acid at position 18 and/or 24 and having 80% or higher homology to the portions of the amino acid sequence represented by SEQ ID NO: 1, outside of the positions 18 and 24,
(7) an amino acid sequence differing from the amino acid sequence represented by SEQ ID NO: 1 by having any amino acid selected from phenylalanine (F), tryptophan (W), histidine (H), and methionine (M) at position 18, or
(8) an amino acid sequence derived from the amino acid sequence represented by SEQ ID NO: 1, having any amino acid selected from phenylalanine (F), tryptophan (W), histidine (H), and methionine (M) at position 18 and having 80% or higher homology to the portions of the amino acid sequence represented by SEQ ID NO: 1, outside of the position 18.

Alternatively, the Affibody used in the present invention may have
(9) an amino acid sequence differing from the amino acid sequence represented by SEQ ID NO: 1 by having any amino acid selected from phenylalanine (F), tryptophan (W), histidine (H), and methionine (M) at position 18 and lacking one to five N-terminal or C-terminal amino acids of SEQ ID NO: 1.

In particular, the amino acid sequence may lack one to five N-terminal and C-terminal amino acids of SEQ ID NO: 1.

The set of photoreceptor protein and Affibody according to the present invention can accurately control complex formation and dissociation thereof through a simple process of light irradiation and shielding.

The wavelength of the light for use when the Affibody and the photoreceptor protein form a complex in a light-dependent manner is in the range of 600 to 750 nm.

A suitable wavelength is set from the states of existence, i.e., the state in which the photoreceptor protein binds to the Affibody in response to light and the state in which the photoreceptor protein does not bind to the Affibody. In order to create a photo state which is the state in which the photoreceptor protein binds to the Affibody, the lower limit value in the range of the wavelength can be 620 nm or more, 630 nm or more, 640 nm or more, 650 nm or more, 660 nm or more, 670 nm or more, 680 nm or more, 690 nm or more, or 695 nm or more, and the upper limit value can be 740 nm or less, 730 nm or less, 720 nm or less, 710 nm or less, or 705 nm or less.

Preferably, the wavelength is in the range of 640 to 720 nm.

In the set of photoreceptor protein and Affibody according to the present invention, the Affibody and the photoreceptor protein form a complex in a light-dependent manner, while the complex formation may be reversibly canceled in a light-dependent manner.

A suitable wavelength is set from the states of existence, i.e., the state in which the photoreceptor protein binds to the Affibody in response to light and the state in which the photoreceptor protein does not bind to the Affibody. In order to create a dark state which is the state in which the photoreceptor protein does not bind to the Affibody, for example, light irradiation may be performed at a near-infrared light wavelength of 750 nm or more, or the set of photoreceptor protein and Affibody may be placed in the dark.

The lower limit value in the range of the wavelength can be 760 nm or more, 770 nm or more, 780 nm or more, or 790 nm or more, and the upper limit value can be 820 nm or less or 810 nm or less.

The set of photoreceptor protein and Affibody according to the present invention forms a complex or dissociates depending on a particular wavelength. The set of photoreceptor protein and Affibody according to the present invention provides a technique that permits precise control and manipulation by red light because the Affibody and the photoreceptor protein form a complex in a red light-dependent manner. The set of photoreceptor protein and Affibody of the present invention which can be applied to optical manipulation of various proteins is referred to as "MagRed".

MagRed is a technique of freely controlling proteins related to vital phenomena or the treatment of diseases by light.

MagRed is capable of providing a technique that permits optical control without adding a cofactor in mammalian cells by using red light in the optical control.

Hereinafter, description will be made in the present specification by describing "MagRed" as the set of Affibody and photoreceptor protein.

MagRed has photoreceptor protein and Affibody. A domain is bound to each of them so that ON/OFF of an effect based on the domain can be accurately controlled by light irradiation and shielding.

The domain to be bound to each of the photoreceptor protein and the Affibody is not particularly limited and can be a domain that exerts its effect by optical control.

The domain that exerts its effect by optical control may be bound to both or one of the photoreceptor protein and the Affibody.

When the domain that exerts its effect by optical control is bound to both of the photoreceptor protein and the Affibody, the photoreceptor protein and the Affibody form a complex by optical control so that the domain bound to the photoreceptor protein and the domain bound to the Affibody can exert their effects in proximity. The domain bound to the photoreceptor protein and the domain bound to the Affibody may be separate domains having different effects, or the domain bound to the photoreceptor protein and the domain bound to the Affibody may exert one effect when located adjacently.

When the domain bound to the photoreceptor protein and the domain bound to the Affibody are separate domains having different effects, these two domains may exert the effects when located adjacently. Examples thereof include heterodimers. Alternatively, the domain bound to the photoreceptor protein and the domain bound to the Affibody may form a homodimer.

Alternatively, a portion of domains that exert an effect by forming a multimer may be used as the domain bound to the photoreceptor protein, and the remaining portion of the domains may be used as the domain bound to the Affibody.

Examples of the case where the domain bound to the photoreceptor protein and the domain bound to the Affibody exert one effect when located adjacently include one domain split into two. In this case, a protein having a certain effect is split into two. When one of the two into which the protein is split is used as the domain bound to the photoreceptor protein and the other half is used as the domain bound to the Affibody, the respective domains bound to the photoreceptor protein and the Affibody form a conjugate by optical control upon which the domain split into two is capable of exerting a function similar to that of the original domain before split.

When the domain that exerts its effect by optical control is bound to one of the photoreceptor protein and the Affibody, a recognition domain that orients a conjugate to the desired location may be bound to the other (photoreceptor protein or Affibody) to which the domain that exerts its effect by optical control is not bound.

The recognition domain bound to one of the photoreceptor protein and the Affibody orients the photoreceptor protein or the Affibody bound by the recognition domain to the desired location.

The photoreceptor protein or the Affibody oriented to the desired location binds to the other (photoreceptor protein or Affibody) by optical control so that the domain bound to the other may exert its effect.

The recognition domain that orients a conjugate to the desired location is not particularly limited, and a domain that recognizes a nucleic acid is suitably used.

In the case of using a domain that recognizes a nucleic acid, MagRed is oriented to a predetermined position through the domain that recognizes a nucleic acid. As a result, the domain that exerts its effect by optical control is also oriented to the predetermined location and exerts its effect.

A domain other than the domain to be bound to each of the photoreceptor protein and the Affibody may be used for allowing the domain bound to each of the photoreceptor protein and the Affibody to exert its effect.

For example, the domains bound to both of the photoreceptor protein and the Affibody may reside in proximity by optical control and exert their effects through an additional domain, or the domain bound to one of the photoreceptor protein and the Affibody may exert its effect through an additional domain bound to neither the photoreceptor protein nor the Affibody.

The binding of the photoreceptor protein and the Affibody to the additional domain is not particularly limited and can be covalent binding. The binding of the photoreceptor protein and the Affibody to the additional domain may be binding via a linker.

A plurality of domains may be bound to the photoreceptor protein or the Affibody, and a plurality of domains may be linked to each other and bound to the photoreceptor protein or the Affibody. When a plurality of domains are linked to each other, the binding between the domains may also be binding via a linker.

A linker known in the art may be used as the linker.

For example, a linker having an amino acid sequence rich in glycine and serine is suitably used as the linker. A linker composed mainly of glycine can be used, and a linker containing a hydrophilic amino acid serine or threonine is suitable. The linker is not particularly limited and may be constituted by 2 to 16 amino acids.

For example, a nuclear localization signal sequence and a 2A peptide sequence or an IRES sequence may be bound as other domains.

Examples of the domain that exerts its function by optical control include, but are not particularly limited to, nuclease, recombinase, polymerase, deaminase, protease, kinase, phosphatase, antibodies and antibody-like molecules, and neurotoxin.

The set of the present invention may be a set of photoreceptor protein and Affibody, wherein
a domain related to the transcriptional activity of a gene is bound to the Affibody, and
a nucleic acid recognition domain is bound to the photoreceptor protein.

The present invention can provide a method for suppressing or activating the expression of a target gene,
the method comprising the step of incubating the target gene and the set of photoreceptor protein and Affibody according to the present invention by light irradiation.

The present invention can also provide a method for suppressing or activating the expression of a target gene, the method comprising the step of incubating the target gene, the set of photoreceptor protein and Affibody according to the present invention, and CRISPR-related protein by light irradiation.

A domain that recognizes a nucleic acid near the target gene is bound to one member of the set of photoreceptor protein and Affibody according to the present invention so that the set of photoreceptor protein and Affibody according to the present invention forms a conjugate by light irradiation, through which the expression of the target gene can be suppressed or activated by the action of a domain related to the transcriptional activity of a gene, which is bound to the other member of the set of photoreceptor protein and Affibody according to the present invention.

A domain that recognizes CRISPR-related protein, preferably guide RNA containing MS2 aptamer in stem loop 2 of tracrRNA, is used in one member of the set of photoreceptor protein and Affibody according to the present invention. By the binding of the MS2 aptamer as a nucleic acid recognition domain, the MS2 aptamer-bound member of the set of photoreceptor protein and Affibody according to the present invention is located near the target gene recognized by the CRISPR-related protein. The set of photoreceptor protein and Affibody according to the present invention forms a conjugate by light irradiation, through which the expression of the target gene can be suppressed or activated by the action of a domain related to the transcriptional activity of a gene, which is bound to the other member of the set of photoreceptor protein and Affibody according to the present invention.

A conventional protein known in the art for use in gene editing is used as the CRISPR-related protein, and other components such as guide RNA may be included together with the CRISPR-related protein. For example, a protein lacking nuclease activity and nickase activity is preferably used as the CRISPR-related protein. The guide RNA is allowed to have a sequence complementary to a gene near the target gene. The set of photoreceptor protein and Affibody according to the present invention is thereby located near the target gene so that the expression of the target gene can be suppressed or activated.

When a functional domain as the domain related to the transcriptional activity of a gene is a transcriptional activation domain or a transcriptional suppression domain, the expression of the target gene is activated or suppressed.

As used herein, the term "expression of a gene" is used to conceptually include both of transcription to synthesize RNA with DNA as a template and translation to synthesize a polypeptide based on the RNA sequence.

VP64, MS2, p65, and HSF1 may be used as functional domains, and a transcriptional activation domain and aptamer binding protein known in the art may be used as functional domains equivalent thereto. For example, a transcriptional activation domain and aptamer binding protein as disclosed in Nature (2015) 517, 583-588 and Nature protocols (2012) 7 (10), 1797-1807 can be used.

The set of the present invention may be a set wherein
one of two fragments into which a protein having a predetermined effect is split binds to the Affibody, and
the other of the two fragments into which the protein having a predetermined effect is split binds to the photoreceptor protein.

The protein split into two is not particularly limited as long as the protein exerts a function by the binding of the photoreceptor protein and the Affibody.

Nonlimiting examples of the protein to be split into two include Cre protein.

A conventional protein known in the art may be used as the Cre protein split into two. Examples thereof include proteins as described in International Publication No. WO 2016/167300.

The Cre protein split into two is not particularly limited as long as two fragments thereof are capable of exerting a function by the binding of the photoreceptor protein and the Affibody. The fragments are constituted by an N-terminal fragment and a C-terminal fragment of the Cre protein.

The split position of the Cre protein is capable of appropriately adopting a conventional split position known in the art.

In the present invention, the N-terminal fragment and the C-terminal fragment of the Cre protein bind to the photoreceptor protein and the Affibody, respectively.

In the set of photoreceptor protein and Affibody according to the present invention, the photoreceptor protein and the Affibody form a conjugate by light irradiation so that the N-terminal fragment of the Cre protein and the C-terminal fragment of the Cre protein bound thereto are reconstituted as the Cre protein to restore the recombinase activity of the Cre protein. Accordingly, the N-terminal fragment of the Cre protein and the C-terminal fragment of the Cre protein can be used as the set of MagRed bound to the Cre protein in light-dependent gene recombination.

In the case of using the N-terminal fragment of the Cre protein and the C-terminal fragment of the Cre protein in gene recombination using the Cre protein, a gene recombination technique by a Cre/loxP system is preferred.

Specifically, gene recombination can be performed in a light-dependent manner by using MagRed bound to the N-terminal fragment of the Cre protein and the C-terminal fragment of the Cre protein in combination with a loxP sequence or a loxP mutant sequence. A nucleic acid having the sequence of any member selected from lox2722, lox66, lox71, JT15, and JTZ17 can be used as the loxP mutant.

The present invention also provides an animal having MagRed.

The tissue penetration of light is important for developing a medical technique based on optical manipulation. MagRed is controllable by red light (up to 660 nm) and near-infrared light (up to 800 nm) having high tissue penetration and is therefore highly advantageous.

The animal having MagRed may have a domain bound to the MagRed and may have a domain bound to the MagRed as well as an additional domain that functions together with the domain.

The animal may have MagRed as a combination of the MagRed, as a nucleic acid encoding at least the MagRed, or as at least one expression vector containing the MagRed.

The animal having MagRed is optically controllable by red light and therefore has gene recombination activity even *in vivo.* Gene recombination can be efficiently controlled by the ex *vivo* light irradiation of the animal in which MagRed optically controllable by red light is incorporated in the body.

Specifically, MagRed can accurately control gene recombination not only *in vitro* but *in vivo* by ON/OFF of light irradiation.

The animal in which MagRed is incorporated is not particularly limited as long as the animal is a mammal. A rodent as a laboratory animal is preferred.

By the incorporation of the MagRed according to the present invention, in the animal having the MagRed, for example, the function of a gene can be knocked out by light stimulation; a mutated gene can be rather restored to normal by light stimulation and evaluated for its influence; or a reporter gene such as a fluorescent protein can be allowed to function by light stimulation so as to label tissues or cells constituting the tissues, though the approach is not particularly limited thereto.

MagRed can be expressed in an animal or cells by selecting respective nucleic acids corresponding to the photoreceptor protein and the Affibody, selecting nucleic acids corresponding to domains, etc. to be bound to the photoreceptor protein and the Affibody, linking the respective nucleic acids such that the photoreceptor protein with the desired domain and the Affibody with the desired domain are expressible, and introducing the respective nucleic acids to an expression vector such as a plasmid, for example.

The present invention also provides a set of nucleic acids designed such that the photoreceptor protein with the desired domain and the Affibody with the desired domain are expressible.

The nucleic acid designed such that the photoreceptor protein with the desired domain is expressible and the nucleic acid designed such that the Affibody with the desired domain is expressible may reside on one expression vector. The respective nucleic acids may reside on the expression vector such that the nucleic acids are linked, or may reside on the expression vector such that their expressions are separately controlled. Alternatively, the nucleic acid designed such that the photoreceptor protein with the desired domain is expressible and the nucleic acid designed such that the Affibody with the desired domain is expressible may reside on separate expression vectors. Such a nucleic acid may be designed such that the photoreceptor protein or the Affibody and the desired domain are connected via a linker.

In the present invention, each nucleic acid can be inserted directly, after digestion with restriction enzymes, or after addition of a linker to downstream of a promoter of an expression vector.

Examples of the expression vector include, but are not particularly limited to, E. coli-derived plasmids (pBR322, pBR325, pUC12, pUC13, pUC18, pUC19, pUC118, pBluescript II, etc.), *Bacillus* subtilis-derived plasmids (pUB110, pTP5, pC1912, pTP4, pE194, pC194, etc.), yeast-derived plasmids (pSH19, pSH15, YEp, YRp, YIp, YAC, etc.), bacteriophages (λ phage, M13 phage, etc.), viruses (retrovirus, vaccinia virus, adenovirus, adeno-associated virus (AAV), cauliflower mosaic virus, tobacco mosaic virus, baculovirus, etc.), and cosmids.

The promoter can be appropriately selected depending on the type of a host. When the host is animal cells, for example, SV40 (simian virus 40)-derived promoter or CMV (cytomegalovirus)-derived promoter can be used. When the host is *E. coli,* for example, trp promoter, T7 promoter, or lac promoter can be used.

A DNA replication origin (ori), a selective marker (antibiotic resistance, auxotrophy, etc.), an enhancer, a splicing signal, a poly-A addition signal, a nucleic acid encoding a tag (FLAG, HA, GST, GFP, etc.), or the like may be incorporated in the expression vector.

In the present invention, a transformant can be obtained by transforming appropriate host cells with the expression vector.

The host can be appropriately selected in relation to the vector. For example, *E. coli, Bacillus subtilis,* a bacterium of the genus *Bacillus,* a yeast, an insect or insect cells, or animal cells can be used. For example, HEK293T cells, CHO cells, COS cells, myeloma cells, HeLa cells, or Vero cells may be used as the animal cells. The transformation can be performed in accordance with a method known in the art such as lipofection, calcium phosphate method, electroporation, microinjection, or particle gun method depending on the type of the host.

The transformant is cultured in accordance with a routine method and thereby expresses the polypeptide of interest.

For protein purification from the cultures of the transformant, the cultured cells are recovered, suspended in an appropriate buffer solution, and disrupted by a method such as sonication or freezing-thawing, and crude extracts are obtained by centrifugation or filtration. When the polypeptide is secreted into a culture solution, a supernatant is recovered.

Purification from the crude extracts or the culture supernatant can also be performed by a method known in the art or a method equivalent thereto (e.g., salting-out, dialysis, ultrafiltration, gel filtration, SDS-PAGE, ion exchange chromatography, affinity chromatography, or reverse-phase high-performance liquid chromatography).

### Examples

### Preparation of expression vector

All constructs for the expression of RedPA-Cre and Red-CPTS were cloned into pcDNA3.1 or pcDNA3.1/V5-His vectors. All constructs related to a TetR-tetO system were cloned into pSV40 vectors. All constructs encoding sgRNA were cloned into pSPgRNA vectors having U6 promoter (Addgene plasmid #47108). Codon-optimized cDNA encoding *Streptococcus pyogenes* Cas9 was amplified from pX330-U6-Chimeric_BB-CBh-hSpCas9 (Addgene plasmid #42230). In order to eliminate the nuclease activity of Cas9, D10A and H840A mutations were introduced to Cas9. cDNA encoding a MS2(N55K) mutant and p65-HSF1 was amplified from MS2-P65-HSF1_GFP (Addgene plasmid #61423). EGFP-Cre gene was amplified from pENN.AAV.hSyn.HI.eGFP-Cre.WPRE.SV40 (Addgene plasmid #105540). cDNA encoding Cre recombinase was synthesized at Invitrogen Corp. cDNA encoding DrBphP, RpBphP1, or PpsR2 was synthesized at GenScript. cDNA (nanoReD2) encoding Zdk1 and LDB-14 was synthesized at Eurofins Genomics. cDNA encoding VP16-NLS and tetR was amplified from tetR-VP16-NLS/pSV40.

### Preparation of ribosome display library of Affibody

Synthetic genes encoding an Affibody library were constructed by PCR reaction at three stages followed by ligation with sequence elements necessary for ribosome display. dsDNA encoding the first and second helices of the Affibody was assembled by annealing of primers O1 and O2 having a complementary 3' end and enzymatic extension. Residues randomized using codons NNK (N = G, A, C, and T; K = G and T) were encoded. Next, overlap extension PCR was performed using primers O3 and O4 so that the third helix was added with the initial reaction product as a template. In the third PCR, the resulting dsDNA encoding the Affibody library was amplified by PCR using primers O5 and O6 and using T7 promoter. In parallel therewith, a nucleotide sequence containing a SecM stalling sequence (AKFSTPVWISQAQGIRAGPQRLT) was amplified with pDgIIISecM-PURF1 as a template by PCR using primers O7 and O8. These two fragments were digested with Type IIS restriction enzyme BsmBI (NEB), and the resulting fragments were then ligated with each other. In order to avoid overamplification or errors in PCR, KOD -Multi&Epi-DNA polymerase (Toyobo Co., Ltd.) was used to set the number of cycles in each PCR step to 5 or less cycles. Each reaction product and ligation product were separated by gel electrophoresis and purified using PureLinkPCR Purification Kit (Invitrogen Corp.), followed by subsequent reaction.

### Selection of Affibody by ribosome display

A sequence encoding DrBphP-PSM was cloned into pColdI vector (Takara Bio Inc.) containing Avi tag following N-terminal His6 tag. *E. coli* C41 (DE3) strain (Lucigen, Wisconsin, USA) carrying pKT270 was transformed with pColdI-Avi-DrBphP-PSM. The transformed cells were cultured at 37°C in 30 ml of LB medium supplemented with ampicillin (100 mg/ml) and chloramphenicol (30 mg/ml) while shaken at 200 rpm until OD590 = 0.45. The cells were cooled to room temperature. Next, isopropyl-β-D-thiogalactoside (IPTG, FUJIFILM Wako Pure Chemical Corp.) and biliverdin (BV, Frontier Scientific Inc.) were added at final concentrations of 0.5 mM and 100 µM, respectively, to the culture solution. The resultant was cultured in the dark at 16°C for 48 hours for protein expression. The cultured cells were recovered by centrifugation and lysed at room temperature for 10 minutes using 0.2 mg of lysozyme (FUJIFILM Wako Pure Chemical Corp.), 10 units of DNase I (Thermo Fisher Scientific Inc.), and 2 ml of B-PER reagent (Thermo Fisher Scientific Inc.) containing protease inhibitor cocktail (Sigma-Aldrich Co., LLC). Crude soluble extracts were purified using HisTrap FF column (GE Healthcare). DrBphP-PSM was biotinylated using BirA enzyme (BirA500, Avidity LLC) in accordance with manufacturer's protocol.

The ribosome-protein fusion library of the Affibody was produced using a PURE system. A standard PURE translation mixture was prepared as mentioned above. *In vitro* transcription and translation reaction was performed at 37°C for 60 minutes. Then, the reaction was terminated with 100 µL of TBS-Mg²⁺ buffer solution (50 mM Tris-HCl, 50 mM MgCl2, and 150 mM NaCl, pH 7.5). Meanwhile, a 30 µL volume of streptavidin magnetic beads (Dynabeads M-280 Streptavidin, Invitrogen Corp.) was washed once with 200 µL of TBS-Mg²⁺ buffer solution. The biotinylated DrBphP-PSM protein was added to the washed beads, which were then incubated at 4°C for 30 minutes while mildly rotated. Then, the incubated beads were washed once with 200 µL of TBS-Mg²⁺ buffer solution. Subsequently, the reaction mixture obtained through *in vitro* transcription and translation reaction was diluted and added thereto. Then, the obtained mixed solution was placed under a light condition of 660 nm or 760 nm so that DrBphP-PSM assumed a photo-state (Pfr) form or a dark-state (Pr) form. Further, the beads were incubated at room temperature for 45 minutes while mildly rotated, and then washed twice with 200 µL of TBS-Mg²⁺ buffer solution. In order to recover the concentrated ribosome-protein fusion product, 200 µL of an elution buffer solution (TBS buffer solution + 50 mM EDTA) was added to the beads, which were then incubated at room temperature for 30 minutes while mildly rotated. The obtained eluate was subjected to standard phenol-chloroform extraction and isopropanol precipitation to remove protein components. The purified RNA was reverse-transcribed using SuperScript III (Thermo Fisher Scientific Inc.) and a primer O8, and the concentrated cDNA library was regenerated by PCR using KOD -Multi&Epi- DNA polymerase and primers O5 and O8. After six repetitive selections, the concentrated DNA library was sequenced (150 cycles, 130 bp and 45 bp, paired ends) in Illumina MiSeq machine using Miseq Reagent Kit v. 3. The results are shown in Figure 6.

Table 1 shows the amino acid sequences (SEQ ID NOs: 41 to 50) of typical Affibody molecules obtained by six selections. In Table 1, the amino acid sequence of No. 6 is identical to the amino acid sequence represented by SEQ ID NO: 1.

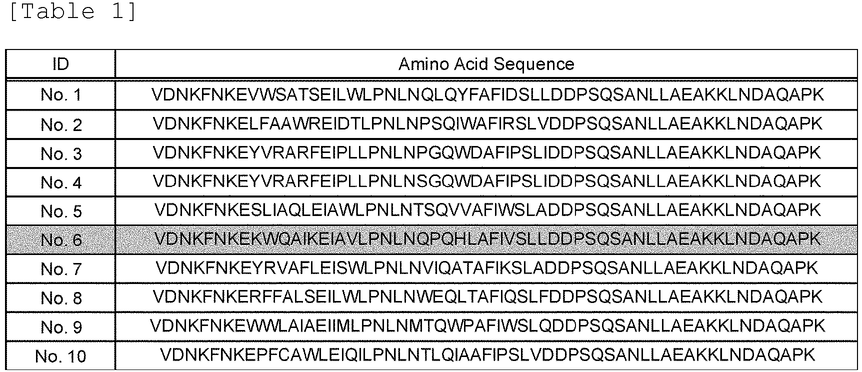

### Analysis using next-generation sequencer

Among the paired-end reads, only R1 reads were subject to subsequent analysis. Sequence analysis was conducted using customed python scripts. For further quality evaluation, two correct sequences in the constant region of the Affibody was searched for. Whether the length between these two sites included exactly 122 nucleotides was evaluated. The sequence having the expected length was considered complete Affibody. Finally, the sequence of Affibody that was concentrated at a wavelength of 660 nm and was not detected at a wavelength of 760 nm was selected.

### Cell culture

HEK 293T cells and Neuro2a cells (ATCC) were cultured at 37°C in 5% CO₂ using Dulbecco's modified Eagle medium (DMEM, Sigma-Aldrich Co., LLC) supplemented with 10% fetal bovine serum (FBS, HyClone), 100 units/mL penicillin, and 100 µg/mL streptomycin (Gibco). HeLa cells (ATCC) were cultured at 37°C in 5% CO₂ using minimal essential medium (Sigma-Aldrich Co., LLC) supplemented with 10% FBS, 100 U/mL penicillin, and 100 µg/mL streptomycin.

### Light source

Light irradiation except for split luciferase assay was performed in a CO₂ incubator. In order to irradiate cells with light, LED arrays (CCS) of 660 nm and 760 nm were placed at the bottom of the incubator, and 24-well and 96-well plates were placed on the LED arrays. A direct-current stabilized power (Kikusui Holdings Corp.) was used in the current control of LED. Time-dependent illumination patterns were prepared using Arduino mega microcontroller board.

### Gene expression system based on TetR-tetO system

HeLa cells were plated at 1.0 × 10⁴ cells per well to a 96-well multiwell plate (Greiner Bio-One International GmbH) in the presence of 25 µM BV and cultured at 37°C in 5% CO₂ for 24 hours. Then, the cells were transfected using Lipofectamine 3000 (Invitrogen Corp.). Plasmids encoding VP16-NLS, tetR, and luciferase reporter were used at a ratio of 1:1:1 in transfection. The total amount of DNA was 0.1 µg per well. Aff6 of Aff6-VP16-NLS was replaced with irrelevant Affibody (Zdk1) as a control condition (Aff6-VP16-NLS (-)). 24 hours after transfection, 100 µL of a medium was added to the sample, followed by light irradiation at 37°C in an environment of 5% CO₂. A red light sample was incubated under a condition of pulsed light illumination (1 W/m²) at 660 nm repetitively involving light irradiation for 1 minute and lights-out for 4 minutes. Under a dark condition, the multiwell plate containing the cultured HeLa cells was covered with aluminum foil and incubated at 37°C in 5% CO₂. After culture for 24 hours, the culture solution was replaced with 100 µL of HBSS (Gibco) containing 500 µM D-luciferin (FUJIFILM Wako Pure Chemical Corp.) as a substrate. Bioluminescence was measured using a luminescence plate reader (Centro XS3 LB 960, Berthold Technologies GmbH & Co. KG) 30 minutes later at room temperature. In the case of using SEAP reporter plasmid, the supernatant of the light-irradiated cells was measured by SEAP reporter gene assay (Roche Diagnostics). The results are shown in Figures 1, 7, and 8.

### Split luciferase assay of MagRed

HEK 293T cells were inoculated at a cell density of 4.0 × 10⁵ cells per well to 35 mm/ Tissue Culture Dish (IWAKI) in the presence of 25 µM biliverdin (BV) added thereto, and cultured at 37°C for 24 hours under a condition of 5% CO₂. The cells were transfected with a plasmid encoding Nfluc-DrBphP and a plasmid encoding 3xFLAG-Aff6_V18FΔN-Cfluc at a ratio of 1:1. The total amount of DNA was 2.5 µg per dish. In the case of using a plasmid encoding full-length firefly luciferase (fluc) as a control, the amount of DNA was 0.025 µg per dish in order to justify a dynamic range of bioluminescence intensity. 24 hours after transfection, the culture solution was replaced with 2 ml of HBSS supplemented with 200 µM D-luciferin. After incubation at room temperature for 30 minutes, DrBphP was optically converted from the photo-state (Pfr) form to the dark-state (Pr) form by irradiation with light (50 W/m²) at 800 nm for 5 minutes using an LED array. Then, bioluminescence was measured at room temperature using GloMax 20/20 luminometer (Promega Corp.). In order to irradiate the cells with light at 660 nm, the bioluminescence measurement was discontinued, and the sample dish was removed from the luminometer. Then, the cells were irradiated with light (10 W/m²) at 660 nm for 1 minute using an LED array of 660 nm. The sample dish was immediately brought back to the luminometer to resume bioluminescence measurement. The procedures of irradiating the cells with light at 800 nm were the same as the procedures mentioned above. The results are shown in Figure 2.

### Luciferase reporter assay of CPTS

HEK 293T cells and Neuro-2a cells at 2.0 × 10⁴ cells and HeLa cells at 1.0 × 10⁴ cells were plated to each well of a 96-well multiwell plate. The cells were cultured at 37°C in 5% CO₂ for 24 hours. The cells were transfected with plasmids encoding NLS-dCas9-NLS, MS2, p65-HSF1, sgRNA, and luciferase reporter at a ratio of 1:1:1:1:1. The total amount of DNA was 0.1 µg per well. Procedures of light irradiation and bioluminescence detection of MagRed were as described above. For a RpBphP1-PpsR2/QPAS1 system, a red light sample was incubated under a condition of pulsed irradiation of 10 W/m² at 760 nm (light irradiation for 1 minute and lights-out for 4 minutes). Other procedures were as described above. Under a condition supplemented with biliverdin (BV) (BV(+)), cells were inoculated in the presence of 25 µM BV and transfected as described above. 24 hours after transfection, 100 µL of a medium supplemented with 25 µM BV was added to the sample. Remaining procedures were as described above. The results are shown in Figure 9. The amino acid sequence and nucleotide sequence of NLS-dCas9-NLS are shown in SEQ ID NOs: 51 and 52. sgRNA is shown in SEQ ID NOs: 53 to 57. SEQ ID NO: 58 represents the MS2 aptamer sequence of sgRNA. In sgRNA, a nucleotide sequence described on the 5'-terminal side from GTTTTA is a target sequence.

The amino acid sequence (SEQ ID NO: 51) of NLS-dCas9-NLS is as described below. The underlined sequences respectively represent the sequences of NLS and dCas9.

MYPYDVPDYASPKKKRKVEASDKKYSIGLAIGTNSVGWAVITDEYKVPSKKFKVLGN
TDRHSIKKNLIGALLFDSGETAEATRLKRTARRRYTRRKNRICYLQEIFSNEMAKVD
DSFFHRLEESFLVEEDKKHERHPIFGNIVDEVAYHEKYPTIYHLRKKLVDSTDKADL
RLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMI
KRYDEHHQDLTLLKALVRQQLPEKYKEIFFDQSKNGYAGYIDGGASQEEFYKFIKPI
LEKMDGTEELLVKLNREDLLRKQRTFDNGSIPHQIHLGELHAILRRQEDFYPFLKDN
REKIEKILTFRIPYYVGPLARGNSRFAWMTRKSEETITPWNFEEVVDKGASAQSFIE
RMTNFDKNLPNEKVLPKHSLLYEYFTVYNELTKVKYVTEGMRKPAFLSGEQKKAIVD
LLFKTNRKVTVKQLKEDYFKKIECFDSVEISGVEDRFNASLGTYHDLLKIIKDKDFL
DNEENEDILEDIVLTLTLFEDREMIEERLKTYAHLFDDKVMKQLKRRRYTGWGRLSR
KLINGIRDKQSGKTILDFLKSDGFANRNFMQLIHDDSLTFKEDIQKAQVSGQGDSLH
EHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSD
YDVDAIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKL
IREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLI
ETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKN

PIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVL
SAYNKHRDKPIREQAENIIHLFTLTNLGAPAAFKYFDTTIDRKRYTSTKEVLDATLI
HQSITGLYETRIDLSQLGGDSPKKKRKVEAS

The nucleotide acid sequence (SEQ ID NO: 52) of NLS-dCas9-NLS is as described below. The underlined sequences respectively represent nucleotide sequences encoding NLS and dCas9.

ATGTACCCATACGATGTTCCAGATTACGCTTCGCCGAAGAAAAAGCGCAAGGTCGAA
GCGTCCGACAAGAAGTACAGCATCGGCCTGGCCATCGGCACCAACTCTGTGGGCTGG
GCCGTGATCACCGACGAGTACAAGGTGCCCAGCAAGAAATTCAAGGTGCTGGGCAAC
ACCGACCGGCACAGCATCAAGAAGAACCTGATCGGAGCCCTGCTGTTCGACAGCGGC
GAAACAGCCGAGGCCACCCGGCTGAAGAGAACCGCCAGAAGAAGATACACCAGACGG
AAGAACCGGATCTGCTATCTGCAAGAGATCTTCAGCAACGAGATGGCCAAGGTGGAC
GACAGCTTCTTCCACAGACTGGAAGAGTCCTTCCTGGTGGAAGAGGATAAGAAGCAC
GAGCGGCACCCCATCTTCGGCAACATCGTGGACGAGGTGGCCTACCACGAGAAGTAC
CCCACCATCTACCACCTGAGAAAGAAACTGGTGGACAGCACCGACAAGGCCGACCTG
CGGCTGATCTATCTGGCCCTGGCCCACATGATCAAGTTCCGGGGCCACTTCCTGATC
GAGGGCGACCTGAACCCCGACAACAGCGACGTGGACAAGCTGTTCATCCAGCTGGTG
CAGACCTACAACCAGCTGTTCGAGGAAAACCCCATCAACGCCAGCGGCGTGGACGCC
AAGGCCATCCTGTCTGCCAGACTGAGCAAGAGCAGACGGCTGGAAAATCTGATCGCC
CAGCTGCCCGGCGAGAAGAAGAATGGCCTGTTCGGCAACCTGATTGCCCTGAGCCTG
GGCCTGACCCCCAACTTCAAGAGCAACTTCGACCTGGCCGAGGATGCCAAACTGCAG
CTGAGCAAGGACACCTACGACGACGACCTGGACAACCTGCTGGCCCAGATCGGCGAC
CAGTACGCCGACCTGTTTCTGGCCGCCAAGAACCTGTCCGACGCCATCCTGCTGAGC
GACATCCTGAGAGTGAACACCGAGATCACCAAGGCCCCCCTGAGCGCCTCTATGATC
AAGAGATACGACGAGCACCACCAGGACCTGACCCTGCTGAAAGCTCTCGTGCGGCAG

CAGCTGCCTGAGAAGTACAAAGAGATTTTCTTCGACCAGAGCAAGAACGGCTACGCC
GGCTACATTGACGGCGGAGCCAGCCAGGAAGAGTTCTACAAGTTCATCAAGCCCATC
CTGGAAAAGATGGACGGCACCGAGGAACTGCTCGTGAAGCTGAACAGAGAGGACCTG
CTGCGGAAGCAGCGGACCTTCGACAACGGCAGCATCCCCCACCAGATCCACCTGGGA
GAGCTGCACGCCATTCTGCGGCGGCAGGAAGATTTTTACCCATTCCTGAAGGACAAC
CGGGAAAAGATCGAGAAGATCCTGACCTTCCGCATCCCCTACTACGTGGGCCCTCTG
GCCAGGGGAAACAGCAGATTCGCCTGGATGACCAGAAAGAGCGAGGAAACCATCACC
CCCTGGAACTTCGAGGAAGTGGTGGACAAGGGCGCTTCCGCCCAGAGCTTCATCGAG
CGGATGACCAACTTCGATAAGAACCTGCCCAACGAGAAGGTGCTGCCCAAGCACAGC
CTGCTGTACGAGTACTTCACCGTGTATAACGAGCTGACCAAAGTGAAATACGTGACC
GAGGGAATGAGAAAGCCCGCCTTCCTGAGCGGCGAGCAGAAAAAGGCCATCGTGGAC
CTGCTGTTCAAGACCAACCGGAAAGTGACCGTGAAGCAGCTGAAAGAGGACTACTTC
AAGAAAATCGAGTGCTTCGACTCCGTGGAAATCTCCGGCGTGGAAGATCGGTTCAAC
GCCTCCCTGGGCACATACCACGATCTGCTGAAAATTATCAAGGACAAGGACTTCCTG
GACAATGAGGAAAACGAGGACATTCTGGAAGATATCGTGCTGACCCTGACACTGTTT
GAGGACAGAGAGATGATCGAGGAACGGCTGAAAACCTATGCCCACCTGTTCGACGAC
AAAGTGATGAAGCAGCTGAAGCGGCGGAGATACACCGGCTGGGGCAGGCTGAGCCGG
AAGCTGATCAACGGCATCCGGGACAAGCAGTCCGGCAAGACAATCCTGGATTTCCTG
AAGTCCGACGGCTTCGCCAACAGAAACTTCATGCAGCTGATCCACGACGACAGCCTG
ACCTTTAAAGAGGACATCCAGAAAGCCCAGGTGTCCGGCCAGGGCGATAGCCTGCAC
GAGCACATTGCCAATCTGGCCGGCAGCCCCGCCATTAAGAAGGGCATCCTGCAGACA
GTGAAGGTGGTGGACGAGCTCGTGAAAGTGATGGGCCGGCACAAGCCCGAGAACATC
GTGATCGAAATGGCCAGAGAGAACCAGACCACCCAGAAGGGACAGAAGAACAGCCGC
GAGAGAATGAAGCGGATCGAAGAGGGCATCAAAGAGCTGGGCAGCCAGATCCTGAAA
GAACACCCCGTGGAAAACACCCAGCTGCAGAACGAGAAGCTGTACCTGTACTACCTG
CAGAATGGGCGGGATATGTACGTGGACCAGGAACTGGACATCAACCGGCTGTCCGAC
TACGATGTGGACGCCATCGTGCCTCAGAGCTTTCTGAAGGACGACTCCATCGACAAC

AAGGTGCTGACCAGAAGCGACAAGAACCGGGGCAAGAGCGACAACGTGCCCTCCGAA
GAGGTCGTGAAGAAGATGAAGAACTACTGGCGGCAGCTGCTGAACGCCAAGCTGATT
ACCCAGAGAAAGTTCGACAATCTGACCAAGGCCGAGAGAGGCGGCCTGAGCGAACTG
GATAAGGCCGGCTTCATCAAGAGACAGCTGGTGGAAACCCGGCAGATCACAAAGCAC
GTGGCACAGATCCTGGACTCCCGGATGAACACTAAGTACGACGAGAATGACAAGCTG
ATCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAGC
GAGTTCGTGTACGGCGACTACAAGGTGTACGACGTGCGGAAGATGATCGCCAAGAGC
GAGCAGGAAATCGGCAAGGCTACCGCCAAGTACTTCTTCTACAGCAACATCATGAAC
TTTTTCAAGACCGAGATTACCCTGGCCAACGGCGAGATCCGGAAGCGGCCTCTGATC
GAGACAAACGGCGAAACCGGGGAGATCGTGTGGGATAAGGGCCGGGATTTTGCCACC
GTGCGGAAAGTGCTGAGCATGCCCCAAGTGAATATCGTGAAAAAGACCGAGGTGCAG
ACAGGCGGCTTCAGCAAAGAGTCTATCCTGCCCAAGAGGAACAGCGATAAGCTGATC
GCCAGAAAGAAGGACTGGGACCCTAAGAAGTACGGCGGCTTCGACAGCCCCACCGTG
GCCTATTCTGTGCTGGTGGTGGCCAAAGTGGAAAAGGGCAAGTCCAAGAAACTGAAG
AGTGTGAAAGAGCTGCTGGGGATCACCATCATGGAAAGAAGCAGCTTCGAGAAGAAT
CCCATCGACTTTCTGGAAGCCAAGGGCTACAAAGAAGTGAAAAAGGACCTGATCATC
AAGCTGCCTAAGTACTCCCTGTTCGAGCTGGAAAACGGCCGGAAGAGAATGCTGGCC
TCTGCCGGCGAACTGCAGAAGGGAAACGAACTGGCCCTGCCCTCCAAATATGTGAAC
TTCCTGTACCTGGCCAGCCACTATGAGAAGCTGAAGGGCTCCCCCGAGGATAATGAG
CAGAAACAGCTGTTTGTGGAACAGCACAAGCACTACCTGGACGAGATCATCGAGCAG
ATCAGCGAGTTCTCCAAGAGAGTGATCCTGGCCGACGCTAATCTGGACAAAGTGCTG
TCCGCCTACAACAAGCACCGGGATAAGCCCATCAGAGAGCAGGCCGAGAATATCATC
CACCTGTTTACCCTGACCAATCTGGGAGCCCCTGCCGCCTTCAAGTACTTTGACACC
ACCATCGACCGGAAGAGGTACACCAGCACCAAAGAGGTGCTGGACGCCACCCTGATC

CACCAGAGCATCACCGGCCTGTACGAGACACGGATCGACCTGTCTCAGCTGGGAGGC
GACAGCCCCAAGAAGAAGAGAAAGGTGGAGGCCAGCTAA

The sequences (SEQ ID NOs: 53 to 57) of sgRNA are as described below. The underlined sequences respectively represent a target sequence and a MS2 aptamer sequence.
P46 sgRNA_Gal4UAS (SEQ ID NO:53) 
GAGTACTGTCCTCCGCGAATTCGTTTTAGAGCTAGGCCAACATGAGGATCACCCATG
TCTGCAGGGCCTAGCAAGTTAAAATAAGGCTAGTCCGTTATCAACTTGAAAAAGTGG
CACCGAGTCGGTGCTTTTTTT
P47 sgRNA_MYOD1 (SEQ ID NO:54) 
GCCTGGGCTCCGGGGCGTTTGTTTTAGAGCTAGGCCAACATGAGGATCACCCATGTC
TGCAGGGCCTAGCAAGTTAAAATAAGGCTAGTCCGTTATCAACTTGAAAAAGTGGCA
CCGAGTCGGTGCTTTTTTT
P48 sgRNA_HBG1 (SEQ ID NO:55) 
GGCTAGGGATGAAGAATAAAGTTTTAGAGCTAGGCCAACATGAGGATCACCCATGTC
TGCAGGGCCTAGCAAGTTAAAATAAGGCTAGTCCGTTATCAACTTGAAAAAGTGGCA
CCGAGTCGGTGCTTTTTTT
P49 sgRNA_IL1R2 (SEQ ID NO:56) 
GTCCTTGGCCACTTCCCCATCGTTTTAGAGCTAGGCCAACATGAGGATCACCCATGT
CTGCAGGGCCTAGCAAGTTAAAATAAGGCTAGTCCGTTATCAACTTGAAAAAGTGGC
ACCGAGTCGGTGCTTTTTTT
P50 sgRNA_ASCL1 (SEQ ID NO:57)

GCAGCCGCTCGCTGCAGCAGGTTTTAGAGCTAGGCCAACATGAGGATCACCCATGTC
TGCAGGGCCTAGCAAGTTAAAATAAGGCTAGTCCGTTATCAACTTGAAAAAGTGGCA
CCGAGTCGGTGCTTTTTTT

### Activation of endogenous gene by Red-CPTS

Procedures of transfection were as described above except that a mixture of sgRNA molecules individually targeting four endogenous genes was used and luciferase reporter was omitted. 48 hours after transfection, total RNA extraction and reverse-transcription PCR were performed using CellAmp Direct RNA Prep Kit (Takara Bio Inc.) and PrimeScript Real-Time Master Mix (Takara Bio Inc.). Quantitative PCR was performed using StepOne Plus system (Thermo Fisher Scientific Inc.) and TaqMan Gene Expression Master Mix (Thermo Fisher Scientific Inc.). The expression levels of the target genes were quantified using TaqMan primers and probes, and GAPDH gene was measured as an endogenous control (Life Technologies Corp.; TaqMan Gene Expression Assay IDs were as follows: ASCL1: Hs04187546_g1, IL1R2: Hs01030384_m1, HBG1: Hs00361131_g1, MYOD1: Hs02330075_g1, GAPDH: Hs99999905_m1). The ΔΔCt method was utilized in order to show each relative mRNA level to a negative control transfected with an empty vector.

### Luciferase reporter assay of RedPA-Cre

HEK 293T cells were inoculated at 2.0 × 10⁴ cells per well to a 96-well multiwell plate in the presence of 25 µM BV and cultured at 37°C in 5% CO₂ for 24 hours. The cells were transfected with plasmids encoding a CreN-containing fusion protein, a CreC-containing fusion protein, and luciferase reporter at a ratio of 1:1:8. The total amount of DNA was 0.1 µg per well. 6 hours after transfection, light irradiation was performed at 37°C under a condition of 5% CO₂. For a MagRed system and a nanoReD1/2 system, a red light sample was incubated under a condition of pulsed irradiation of 1 W/m² at 660 nm (light irradiation for 1 minute and lights-out for 4 minutes). For RpBphP1-PpsR2/QPAS1, a red light sample was incubated under a condition of pulsed irradiation of 10 W/m² at 760 nm (light irradiation for 1 minute and lights-out for 4 minutes). Under a dark condition, the multiwell plate containing the cultured HEK 293T cells was covered with aluminum foil and incubated at 37°C in 5% CO₂. After culture for 18 hours, the culture solution was replaced with 100 µL of HBSS (Gibco) containing 200 µM D-luciferin (FUJIFILM Wako Pure Chemical Corp.) as a substrate. Bioluminescence was measured using a luminescence plate reader (Centro XS3 LB 960, Berthold Technologies GmbH & Co. KG) 30 minutes later at room temperature. The results are shown in Figures 5 and 10 to 14.

### Fluorescent reporter assay of RedPA-Cre

HEK 293T cells were plated at 0.8 × 10⁵ cells/well to a 24-well plate and cultured at 37°C in 5% CO₂ for 24 hours. The cells were transfected with plasmids encoding NLS-CreN104-Aff6_V18FΔN-Venus, NLS-DrBphP(FL)-CreC106, and fluorescent reporter at a ratio of 1:1:8. The total amount of DNA was 0.5 µg per well. 6 hours after transfection, the sample was irradiated with light at 37°C under a condition of 5% CO₂. A red light sample was incubated under a condition of pulsed irradiation of 1 W/m² at 660 nm (light irradiation for 1 minute and lights-out for 4 minutes). Under a dark condition, the multiwell plate containing the cultured HEK 293T cells was covered with aluminum foil and incubated at 37°C in 5% CO₂. After culture for 24 hours, the cells were fixed in PBS containing 4% paraformaldehyde for 15 minutes and then incubated at room temperature for 1 hour in a blocking solution (PBS containing 5% BSA and 0.3% Triton-X100) to perform blocking while enhancing cell membrane permeability. Then, the cells were immunostained while mildly shaken at room temperature for 1 hour in the blocking solution using a mouse anti-mCherry antibody (mCherry Monoclonal Antibody conjugated with Alexa Fluor 594, Invitrogen Corp., Cat# M11240, 1:1,000 dilution). The cells were washed three times with PBS and then stained with 300 nM DAPI (Invitrogen Corp.) at room temperature for 15 minutes. Then, images were obtained using an invert microscope (DMI6000B, Leica Microsystems K.K.) equipped with a 10× objective lens (Leica HCX PL FLUOTAR 10x/0.30NA PH1) and an EM-CCD camera (Cascade II:512, Photometrics, Arizona, USA). DAPI, GFP, and RFP channels were imaged using A, L5, and TX2 filter cubes (Leica Microsystems K.K.). The results are shown in Figures 3 and 4.

### Fluorescence image processing

Fluorescence images obtained in experiments were analyzed using FIJI imageJ and CellProfiler. The background of the images was subtracted using Subtract background tool included in ImageJ. The nuclei were identified using the threshold of an object size at a 5-to 20-pixel unit in a DAPI channel. In order to determine populations of GFP-positive cells (GFP+) and mCherry-positive cells (mCh+), average fluorescence intensity of Venus and mCherry was measured in respective cell nuclei. The threshold of fluorescence intensity was determined such that mCh+ cells were not detected under a control condition. The number of mCh+ cells was divided by the number of GFP+ cells and multiplied by 100 to determine the ratio of mCh+/GFP+ cells.

### Evaluation of RedPA-Cre in mouse in vivo

10 µg of RedPA-Cre plasmid and 40 µg of bioluminescent reporter plasmid were mixed in TransIT-EE Hydrodynamic Delivery Solution (Mirus Bio LLC), and the plasmids were introduced to the liver of each 4-week-old female ICR mouse (Sankyo Labo Service Corporation, Inc.) by hydrodynamic tail vein injection (HTV injection). 8 hours later, the hair on the abdominal surface of the mouse was removed using a hair removal cream. The mice were randomly assigned to a dark group and a red light irradiation group. Then, the mice in the red light irradiation group were irradiated from an LED light source (660 nm; 100 W/m²). The mice were temporarily brought back to home cages every 8 hours and fed for 1 hour. The mice in the dark group were placed in home cages shielded from light. 25 hours after hydrodynamic tail vein injection, bioluminescence was imaged. 200 mL of 100 mM D-luciferin was intraperitoneally injected to each mouse before bioluminescence imaging. Then, the mouse was anesthetized with isoflurane (FUJIFILM Wako Pure Chemical Corp.). 5 minutes after D-luciferin injection, bioluminescence images of the mice were obtained using a bioluminescence imager (Lumazone, Nippon Roper K.K.) equipped with an EMCD camera (Evolve 512, Teledyne Photometrics). The results are shown in Figures 5 and 15.

## Claims

1. A set of Affibody and photoreceptor protein, wherein the Affibody and the photoreceptor protein form a complex in a manner dependent on light at a wavelength of 600 to 750 nm.

2. The set according to claim 1, wherein the Affibody has any of the following amino acid sequences (1) to (4):
(1) the amino acid sequence represented by SEQ ID NO: 1,
(2) an amino acid sequence differing from the amino acid sequence represented by SEQ ID NO: 1 by mutation of an amino acid at one to five positions among positions 9 to 11, 13, 14, 17, 18, 24, 25, 27, 28, 32, and 35,
(3) an amino acid sequence having 80% or higher homology to the portions of the amino acid sequence represented by SEQ ID NO: 1, outside of the positions 9 to 11, 13, 14, 17, 18, 24, 25, 27, 28, 32, and 35, and
(4) an amino acid sequence derived from the amino acid sequence represented by SEQ ID NO: 1, having mutation of an amino acid at one to five positions among positions 9 to 11, 13, 14, 17, 18, 24, 25, 27, 28, 32, and 35 and having 80% or higher homology to the portions of the amino acid sequence represented by SEQ ID NO: 1, outside of the positions 9 to 11, 13, 14, 17, 18, 24, 25, 27, 28, 32, and 35.

3. The set according to claim 1, wherein the Affibody has the following amino acid sequence (5) or (6):
(5) an amino acid sequence differing from the amino acid sequence represented by SEQ ID NO: 1 by mutation of an amino acid at position 18 and/or 24, and
(6) an amino acid sequence derived from the amino acid sequence represented by SEQ ID NO: 1, having mutation of an amino acid at position 18 and/or 24 and having 80% or higher homology to the portions of the amino acid sequence represented by SEQ ID NO: 1, outside of the positions 18 and 24.

4. The set according to claim 1, wherein the Affibody has the following amino acid sequence (7) or (8):
(7) an amino acid sequence differing from the amino acid sequence represented by SEQ ID NO: 1 by having any amino acid selected from phenylalanine (F), tryptophan (W), histidine (H), and methionine (M) at position 18, and
(8) an amino acid sequence derived from the amino acid sequence represented by SEQ ID NO: 1, having any amino acid selected from phenylalanine (F), tryptophan (W), histidine (H), and methionine (M) at position 18 and having 80% or higher homology to the portions of the amino acid sequence represented by SEQ ID NO: 1, outside of the position 18.

5. The set according to claim 1, wherein the Affibody has the following amino acid sequence (9):
(9) an amino acid sequence differing from the amino acid sequence represented by SEQ ID NO: 1 by having any amino acid selected from phenylalanine (F), tryptophan (W), histidine (H), and methionine (M) at position 18 and lacking one to five N-terminal or C-terminal amino acids of SEQ ID NO: 1.

6. The set according to any one of claims 1 to 5, wherein the photoreceptor protein is DrBphP.

7. The set according to any one of claims 1 to 5, wherein the photoreceptor protein
(1) has the amino acid sequence represented by SEQ ID NO: 2, or
(2) has an amino acid sequence having 80% or higher homology to the amino acid sequence represented by SEQ ID NO: 2.

8. The set according to any one of claims 1 to 7, wherein a domain related to the transcriptional activity of a gene is bound to one of the Affibody and the photoreceptor protein, and a nucleic acid recognition domain is bound to the other.

9. The set according to any one of claims 1 to 7, wherein one of two fragments into which a protein having a predetermined effect is split is bound to the Affibody, and the other of the two fragments into which the protein having a predetermined effect is split is bound to the photoreceptor protein.
